(19) Europäisches Patentamt
European Patent Office
Office européen des brevets

(11) EP 2 500 022 B1

(12) **EUROPEAN PATENT SPECIFICATION**

(45) Date of publication and mention of the grant of the patent:
**16.12.2015 Bulletin 2015/51**

(21) Application number: **10829882.9**

(22) Date of filing: **05.11.2010**

(51) Int Cl.:
***A61K 31/4745*** (2006.01) ***A61K 31/4375*** (2006.01)
***A61K 31/4709*** (2006.01) ***A61K 31/496*** (2006.01)
***A61K 31/5383*** (2006.01) ***A61P 31/04*** (2006.01)
***A61P 43/00*** (2006.01) ***C07D 498/16*** (2006.01)
***A61K 31/47*** (2006.01) ***C12Q 1/18*** (2006.01)

(86) International application number:
**PCT/JP2010/069674**

(87) International publication number:
**WO 2011/058923 (19.05.2011 Gazette 2011/20)**

(54) **ANTIBACTERIAL AGENT FOR DRUG-RESISTANT BACTERIA AND USE OF SAME**

ANTIBAKTERIELLES MITTEL FÜR WIRKSTOFFRESISTENTE BAKTERIEN UND VERWENDUNG DAVON

AGENT ANTIBACTÉRIEN CONTRE DES BACTÉRIES PHARMACORÉSISTANTES ET USAGE ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.11.2009 JP 2009260363**

(43) Date of publication of application:
**19.09.2012 Bulletin 2012/38**

(73) Proprietors:
- **Microbial Chemistry Research Foundation Tokyo 141-0021 (JP)**
- **Juntendo Educational Foundation Bunkyo-ku, Tokyo 113-8421 (JP)**

(72) Inventors:
- **IGARASHI, Masayuki Machida-shi Tokyo 194-0031 (JP)**
- **HIRAMATSU, Keiichi Tokyo 106-0032 (JP)**

(74) Representative: **Graf von Stosch, Andreas et al Graf von Stosch Patentanwaltsgesellschaft mbH Prinzregentenstrasse 22 80538 München (DE)**

(56) References cited:
**JP-A- H11 506 101** **JP-A- 2004 532 876**
**JP-A- 2007 126 452**

- **MASAYOSHI ARAI ET AL.: 'Kosei Busshitsu nybomycin no Senzaisei Kekkakukin ni Taisuru Kokin Kassei to sono Sayo Mechanism no Kaiseki' DAI 27 KAI ABSTRACTS OF SYMPOSIUM ON MEDICINAL CHEMISTRY 2008, pages 354 - 355, XP008160344**
- **STRELITZ,F. ET AL.: 'Nybomycin, a new antibiotic with antiphage and antibacterial properties' PROCEEDINGS OF THE NATIONAL ACADEMY OF SCIENCES OF THE UNITED STATES OF AMERICA vol. 41, 1955, XP008156822**
- **NADZAN,A.M. ET AL.: 'Hydroxynybomycin: isolation, structure and bioactivity' THE JOURNAL OF ANTIBIOTICS vol. 30, no. 6, 1977, pages 523 - 524, XP008156820**
- **POPE,J.A. ET AL.: 'Applications of thin layer chromatography, high performance liquid chromatography and mass spectrometry in the fermentation and isolation of the antibiotic nybomycin' JOURNAL OF INDUSTRIAL MICROBIOLOGY vol. 6, no. 1, 1990, pages 61 - 69, XP008156827**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 500 022 B1

## Description

### Technical Field

The present invention relates to an antibacterial agent for use in treating an individual infected with a drug-resistant bacteria and an infectious disease combined drug for use in treating an individual infected with a drug-resistant bacteria, etc.

### Background Art

Since the practical use of the first antibiotic penicillin G in 1940, many antibacterial agents have been developed, and antibacterial chemotherapy has greatly contributed to the advance of modern medicine and the extension of the average lifetime. However, pathogenic bacteria have acquired resistance to such antibacterial agents one after another, and the effects of antibacterial chemotherapy have considerably decreased in the 21st century (see Frontiers in Antimicrobial Resistance. Edited by White, D.G., Alekshun, M. N. and McDermott, P. F. 2005, ASM Press American Society for Microbiology Washington, DC). In particular, in 1960 in the United Kingdom, methicillin-resistant S. aureus (MRSA), which acquired resistance to all β-lactam antibacterial agents, appeared from Staphylococcus aureus (S. aureus) which is a pathogenic bacterium mainly causing hospital infection (see Jevons, M. P., Br Med J.s, 1961, (1). 124-125). Since then, the number of MRSA has continued to increase up to the present, so that it has spread all over the world today. It is not too much to say that there are no hospitals without MRSA.

There are many types of antibacterial agents. Thus, even when one type of antibacterial agent is ineffective to an infectious disease, the infectious disease can be cured using another type of antibacterial agent having a different action mechanism. Pathogenic bacteria, however, have continued to acquire resistance to each of the antibacterial agents. As a result, by the end of the 20th century, multiply antibiotic-resistant bacteria appeared which acquired resistance to almost all currently-available antibacterial agents. Typical examples of the multiply antibiotic-resistant bacteria include MRSA as Gram-positive bacteria, Acinetobacter as Gram-negative bacteria, and tuberculosis bacteria as acid-fast bacteria.

Vancomycin was an only antibacterial agent that maintained its effectiveness to multiply antibiotic-resistant MRSA. The present inventors, however, found out in 1996 vancomycin-intermediate S. aureus (VISA) to which vancomycin therapy was ineffective (see Hiramatsu, K., Hanaki, H., et al., J Antimicrob Chemother, 1997, 40(1), 135-6). Since then, VISA has been found in various countries in the world up to the present. Moreover, vancomycin-resistant S. aureus (VRSA), which is MRSA highly resistant to vancomycin, was found in the U.S. in 2003, and at present, 9 strains have been reported in total from the three countries; i.e., the U.S., Iran and India (see Perichon B, Courvalin P., Antimicrob Agents Chemother, 2009, Nov; 53(11), 4580-7, Epub 2009 Jun 8).

Mu50 is a typical strain of VISA (see Hiramatsu, K., Hanaki, H., et al., J Antimicrob Chemother, 1997, 40(1), 135-6) and is resistant to, for example, β-lactam, tetracycline, minocycline, aminoglycoside, macrolide, rifampin, quinolone and glycopeptide. Also, it is reported that almost all the VISA strains separated from various countries in the world exhibit similar multiply resistance. As described above, S. aureus becomes multiply antibiotic-resistant, and at present, there are no effective antibacterial agents thereagainst.

Nadzah, A.M. et al. (The Journal of Antibiotics, vol. 30(6), 1977, pp. 523-524) report on the isolation, structure and bioactivity of hydroxynybomycin as antibiotic agent.

In general, screening for antibacterial agents has been conducted to explore and select an antibacterial agent effective to both drug-susceptible bacteria and drug-resistant bacteria. However, in view of the above-described events during the past half century, it is apparent that the antibacterial agent effective to both the drug-susceptible bacteria and the drug-resistant bacteria will problematically form resistant bacteria sooner or later, even if it has a different action mechanism to bacteria from the conventional antibacterial agents.

Therefore, keen demand has arisen for the provision of an antibacterial agent for drug-resistant bacteria which exhibits a low antibacterial activity against bacteria acquiring no drug resistance and thus does not permit them to become drug resistant, which exhibits an excellent antibacterial activity against drug-resistant bacteria resistant to at least one drug, which involves no side effects and which can be produced simply; a screening method therefore; an infectious disease therapeutic drug containing the antibacterial agent for drug-resistant bacteria.

### Summary of Invention

### Technical Problem

The present invention solves the above existing problems and aims to achieve the following objects. Specifically, an object of the present invention is to provide an antibacterial agent for use in treating an individual infected with a

drug-resistant bacteria which exhibits a low antibacterial activity against bacteria acquiring no drug resistance and thus does not permit them to become drug resistant, which exhibits an excellent antibacterial activity against drug-resistant bacteria resistant to at least one drug, which involves no side effects and which can be produced simply; an infectious disease therapeutic drug and an infectious disease combined therapeutic drug for use in treating an individual infected with a drug-resistant bacteria each containing the antibacterial agent for drug-resistant bacteria.

Solution to Problem

In order to solve the above existing problems, the present inventors conducted extensive studies and have obtained the following findings. That is, they have found that an antibacterial agent of General Formula (1) for use in treating an individual infected with a drug-resistant bacteria according to the present invention, which is effective to drug-resistant bacteria resistant to at least one drug, preferably can suppress the growth of the drug-resistant bacteria resistant to at least one drug without suppressing the growth of drug-susceptible bacteria; that the antibacterial agent for drug-resistant bacteria exhibits an excellent antibacterial activity against the drug-resistant bacteria derived from various countries of the world; i.e., VISA clinical isolates of MRSA, VISA clinical isolates of MSSA and VISA clinical isolates almost all of which are resistant to quinolones; that the nybomycins exhibit excellent antibacterial activities against quinolone-resistant enterococcus; a (non-claimed) screening method can select an antibacterial agent for drug-resistant bacteria exhibiting an excellent antibacterial activity against drug-resistant bacteria without permitting drug-susceptible bacteria to become resistant. The present invention has been accomplished on the basis of the findings.

The present invention is based on the above findings obtained by the present inventors. Means for solving the above problems are as follows.

<1> An antibacterial agent for use in treating an individual infected with drug-resistant bacteria, wherein the antibacterial agent (nybomycin) is represented by the following General Formula (1):

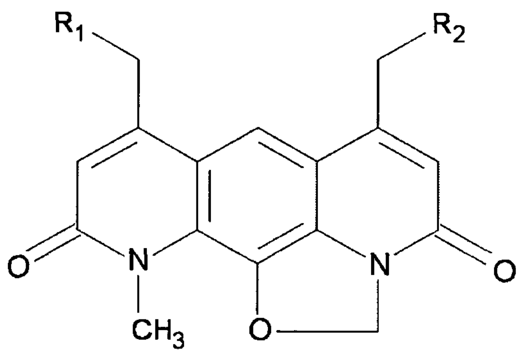

General Formula (1)

where $R_1$ represents OH or H and $R_2$ represents H,
wherein the antibacterial agent for drug-resistant bacteria is effective to drug-resistant bacteria resistant to at least one drug.

<2> The antibacterial agent for use in treating an individual infected with drug-resistant bacteria according to <1>, wherein the antibacterial agent is at least one of nybomycin, deoxynybomycin and derivatives thereof.

<3> The antibacterial agent for use in treating an individual infected with drug-resistant bacteria according to any one of <1> to <2>, wherein the antibacterial agent is at least one of nybomycin and deoxynybomycin.

<4> The antibacterial agent for use in treating an individual infected with drug-resistant bacteria according to any one of <1> to <3>, wherein the antibacterial agent for drug-resistant bacteria has a minimum inhibitory concentration of lower than 8 mg/L against the drug-resistant bacteria.

<5> The antibacterial agent for use in treating an individual infected with drug-resistant bacteria according to any one of <1> to <4>, wherein the drug-resistant bacteria include at least quinolone-resistant bacteria.

<6> The antibacterial agent for use in treating an individual infected with drug-resistant bacteria according to <5>, wherein the quinolone-resistant bacteria each have a mutation in a gene of at least one of DNA gyrase and topoisomerase IV

<7> The antibacterial agent for use in treating an individual infected with drug-resistant bacteria according to any one of <1> to <5>, wherein the antibacterial agent for drug-resistant bacteria is ineffective to at least quinolone-susceptible bacteria.

<8> The antibacterial agent for use in treating an individual infected with drug-resistant bacteria according to <7>, wherein the antibacterial agent for drug-resistant bacteria has a minimum inhibitory concentration of 8 mg/L or higher against the quinolone-susceptible bacteria.
<9> An infectious disease therapeutic drug for use in treating an individual infected with a drug-resistant bacteria including:

the antibacterial agent for drug-resistant bacteria according to any one of <1> to <8>.

<10> The infectious disease therapeutic drug for use in treating an individual infected with a drug-resistant bacteria according to <9>, wherein the infectious disease therapeutic drug is at least one of a MRSA infectious disease therapeutic drug, a VISA infectious disease therapeutic drug and a VRSA infectious disease therapeutic drug.
<11> The infectious disease therapeutic drug for use in treating an individual infected with a drug-resistant bacteria according to <9>, wherein the infectious disease therapeutic drug is an enterococcus infectious disease therapeutic drug.
<12> The infectious disease therapeutic drug for use in treating an individual infected with a drug-resistant bacteria according to <11>, wherein the enterococcus infectious disease therapeutic drug is a therapeutic drug for an infectious disease caused by quinolone-resistant enterococcus.
<13> An infectious disease combined drug for use in treating an individual infected with a drug-resistant bacteria including:

the antibacterial agent for drug-resistant bacteria according to any one of <1> to <8>, and
at least one quinolone,
wherein the antibacterial agent for drug-resistant bacteria is used in combination with the at least one quinolone.

<14> A (non-claimed) screening method for an antibacterial agent for drug-resistant bacteria, the screening method including:

separately culturing quinolone-susceptible bacteria and quinolone-resistant bacteria in presence of a test sample at the same concentration, and
selecting the test sample that does not suppress the growth of the quinolone-susceptible bacteria and suppresses the growth of the quinolone-resistant bacteria.

<15> The screening method according to <14>, wherein the quinolone-susceptible bacteria are quinolone-susceptible Staphylococcus aureus and the quinolone-resistant bacteria are quinolone-resistant Staphylococcus aureus.

Advantageous Effects of Invention

The present invention can provide an antibacterial agent for use in treating an individual infected with drug-resistant bacteria which exhibits a low antibacterial activity against bacteria acquiring no drug resistance and thus does not permit them to become drug resistant, which exhibits an excellent antibacterial activity against drug-resistant bacteria resistant to at least one drug, which involves no side effects and which can be produced simply; an infectious disease therapeutic drug and an infectious disease combined therapeutic drug for use in treating an individual infected with a drug-resistant bacteria each containing the antibacterial agent for drug-resistant bacteria; a (non-claimed) screening method for the antibacterial agent for drug-resistant bacteria. These can solve the above problems to achieve the above objects.

Brief Description of Drawings

Fig. 1 is a chart of an ultraviolet ray absorption spectrum of nybomycin measured in methanol, where the vertical axis: absorbance (Abs) and the horizontal axis: wavelength (nm).
Fig. 2 is a chart of a $^{1}$H-NMR spectrum of nybomycin measured in deuterated trifluoroacetic acid at 25°C and 600 MHz, where the unit of the horizontal axis: ppm.
Fig. 3 is a chart of $^{13}$C-NMR spectrum of nybomycin measured in deuterated trifluoroacetic acid at 25°C and 150 MHz, where the unit of the horizontal axis: ppm.
Fig. 4 is a chart of an ultraviolet ray absorption spectrum of deoxynybomycin measured in methanol, where the vertical axis: absorbance (Abs) and the horizontal axis: wavelength (nm).

Fig. 5 is a chart of a $^{1}$H-NMR spectrum of deoxynybomycin measured in deuterated trifluoroacetic acid at 25°C and 600 MHz, where the unit of the horizontal axis: ppm.
Fig. 6 is a chart of $^{13}$C-NMR spectrum of deoxynybomycin measured in deuterated trifluoroacetic acid at 25°C and 150 MHz, where the unit of the horizontal axis: ppm.

Description of Embodiments

(Antibacterial agent for use in treating an individual infected with drug-resistant bacteria)

An antibacterial agent for use in treating an individual infected with drug-resistant bacteria of the present invention, wherein the antibacterial agent is a compound represented by General Formula (1); and, if necessary, further contains other ingredients.

The antibacterial agent for use in treating an individual infected with drug-resistant bacteria according to the present invention is preferably an antibacterial agent selected by a screening method according to <14>.

<Nybomycin>

The nybomycin may be appropriately selected depending on the intended purpose, but is a compound represented by the following General Formula (1).

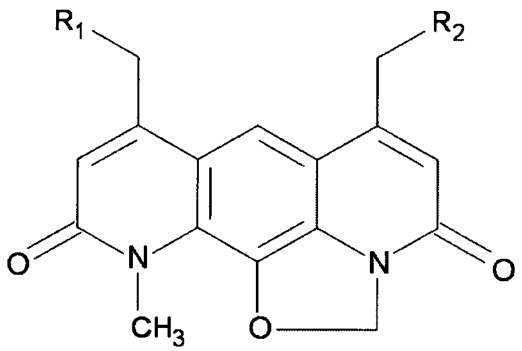

General Formula (1)

In General Formula (1), $R_1$ represents OH or H and $R_2$ represents H.

The compound represented by the above General Formula (1) is a nybomycin having a structure expressed by the following Structural Formula (1) and a deoxynybomycin having a structure expressed by the following Structural Formula (2), since they exhibit a high antibacterial activity. These may be used alone or in combination.

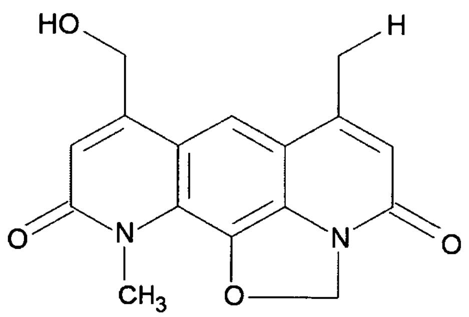

Structural Formula (1)

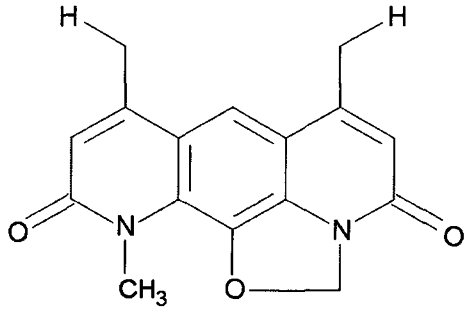

Structural Formula (2)

<<Amount>>

The amount of the at least one of the antibacterial agent of General Formula (1) for use in treating an individual infected with drug-resistant bacteria is not particularly limited and may be appropriately selected depending on the intended purpose. Also, the antibacterial agent for use in treating an individual infected with drug-resistant bacteria may be at least one of the nybomycin of General Formula (1).

<<Production method>>

The method for producing at least one of the antibacterial agent of General Formula (1) and the derivative thereof is not particularly limited and may be appropriately selected from known methods. Examples of the method include a method for producing it from microorganisms that produce at least one of the nybomycin of General Formula (1) and the derivative thereof, a method for producing it based on genetically engineering techniques, and a method for producing it through chemical synthesis (see Strelitz F, et al., PNAS, 1955, 41(9), 620-624.; Rinehart KL Jr, et al., J Am Chem Soc, 1970, 92(23), 6994-6995.; Naganawa H, et al., J Antibiot, 1970, 23(7), 365-368.).

The method for producing at least one of the antibacterial agent of General Formula (1) and the derivative thereof from microorganisms that produce the at least one of the antibacterial agent of General Formula (1) and the derivative thereof is not particularly limited and may be appropriately selected depending on the intended purpose. Examples of the method include a method including culturing microorganisms that produce at least one of the antibacterial agent of General Formula (1) and the derivative thereof, and recovering the at least one of the antibacterial agent of General Formula (1) and the derivative thereof from the microorganisms.

The above microorganisms (hereinafter may be referred to as "nybomycin-producing microorganisms") are not particularly limited, as long as they can produce at least one of the antibacterial agent of General Formula (1) and the derivative thereof, and may be appropriately selected depending on the intended purpose. Examples of the microorganisms include microorganisms belonging to the genus Streptomyces. Among others, the nybomycin-producing bacteria are preferably Streptomyces hyalinum-MB891-A1 strain (ATCC29817).

Also, other microorganisms that can produce at least one of the antibacterial agent of General Formula (1) and the derivative thereof can be separated from the natural world by a routine method.

Notably, when the microorganisms that can produce at least one of the antibacterial agent of General Formula (1) and the derivative thereof, including the above Streptomyces hyalinum strain, are irradiated with radioactive rays or subjected to other mutation treatments, the ability to produce at least one of the antibacterial agent of General Formula (1) and the derivative thereof can be increased. Also, genetically engineering techniques may be used to increase the ability to produce at least one of the antibacterial agent of General Formula (1) and the derivative thereof.

The above culturing is performed by inoculating the nybomycin-producing microorganisms into a nutrient medium (hereinafter may be referred to simply as "medium") and culturing the nybomycin-producing microorganisms at a temperature suitable to the production of at least one of the nybomycin of General Formula (1) and the derivative thereof.

The nutrient medium is not particularly limited and may be appropriately selected depending on the intended purpose. Examples of the nutrient medium usable include known nutrient media conventionally used for culturing actinomycetes.

The nutrient sources to be added to the nutrient medium are not particularly limited and may be appropriately selected depending on the intended purpose. The nitrogen source may be, for example, soy flour, peptone, yeast extract, meat extract, corn steep liquor and ammonium sulfate. The carbon source may be, for example, carbohydrates (e.g., tomato paste, glycerin, starch, glucose, galactose and dextrin) and fats. Furthermore, inorganic salts such as sodium chloride and calcium carbonate may be added to the medium. If necessary, a trace amount of a metal salt may be added

to the medium.

Any known material used for culturing may be used so long as the nybomycin-producing microorganisms may utilize them to produce antibacterial agent of General Formula (1).

The seed culture liquid for producing at least one of the antibacterial agent of General Formula (1) and the derivative thereof is not particularly limited and may be appropriately selected depending on the intended purpose. For example, the seed culture liquid may be a product obtained through slant culture of the nybomycin-producing microorganisms on an agar medium.

The culture conditions are not particularly limited and may be appropriately selected depending on the intended purpose, but are preferably aerobic conditions.

The culture temperature is not particularly limited, so long as it is in a range within which the growth of the nybomycin-producing microorganisms is not substantially inhibited as well as the nybomycin-producing microorganisms can produce at least one of the nybomycin of General Formula (1) and the derivative thereof, and may be appropriately selected depending on, for example, the type of the nybomycin-producing microorganisms. The culture temperature is preferably 25°C to 35°C.

The culture period is not particularly limited and may be appropriately selected in consideration of the amount of at least one of the antibacterial agent of General Formula (1) and the derivative thereof accumulated. In general, the amount of at least one of the antibacterial agent of General Formula (1) and the derivative thereof accumulated becomes maximal for a culture period of 3 days to 10 days.

The method for recovering at least one of the antibacterial agent of General Formula (1) and the derivative thereof from the nybomycin-producing microorganisms is not particularly limited and may be appropriately selected depending on, for example, physico-chemical properties of at least one of the antibacterial agent of General Formula (1) and the derivative thereof. For example, the recovering method usable may be a known method for recovering metabolites produced by microorganisms.

Specific examples of the recovering method include a method in which the culture supernatant obtained at the culturing is extracted with a solvent that is not miscible in water; a method utilizing the difference in adsorption affinity to various adsorbents; a method using gel filtration; and a chromatography method utilizing counter-current distribution. These may be used alone or in combination.

Alternatively, the microorganisms may be separated and treated by, for example, an extraction method using an appropriate organic solvent or an elution method involving homogenizing microorganisms to extract at least one of the antibacterial agent of General Formula (1) and the derivative thereof, which is then isolated and purified in the same manner as described above for recovery.

The structure of the isolated and purified product of at least one of the antibacterial agent of General Formula (1) and the derivative thereof can be confirmed by various analysis methods appropriately selected. The analysis method can be performed by analyzing, for example, mass spectrum, UV absorption spectrum, proton nuclear magnetic resonance spectrum and $^{13}C$ nuclear magnetic resonance spectrum.

<Other ingredients>

The other ingredients are not particularly limited and may be appropriately selected depending on the intended purpose. Examples thereof include pharmacologically acceptable carriers such as ethanol, water and starch.

The amount of the other ingredients contained in the antibacterial agent for use in treating an individual infected with a drug-resistant bacteria is not particularly limited and may be appropriately selected depending on the intended purpose from a range within which the effects of at least one of the antibacterial agent of General Formula (1) and the derivative thereof are not impeded.

<Target>

The target against which the antibacterial agent for use in treating an individual infected with a drug-resistant bacteria exhibits an antibacterial activity is not particularly limited, as long as it is drug-resistant bacteria that are resistant to at least one drug, and may be appropriately selected depending on the intended purpose. The target is preferably quinolone-resistant bacteria, more preferably multiply antibiotic-resistant bacteria.

<<Quinolone-resistant bacteria»

The quinolones are synthetic antibacterial drugs inhibiting DNA synthesis of bacteria by inhibiting the activity of at least one of DNA gyrase and topoisomerase IV (hereinafter may be referred to simply as "topoisomerase") and have widely been used as pharmaceutical drugs and drugs for animals.

The quinolones are classified into the first-generation quinolones (old quinolones) and the second-generation

quinolones (new quinolones).

The old quinolones exhibit an antibacterial activity against Gram-negative bacteria and thus are used for infectious diseases in the urinary tract and the genital, intestinal infectious diseases, and the like. However, the old quinolones exhibit a low antibacterial activity against Gram-positive bacteria and thus cannot be used for respiratory infectious diseases caused by, for example, pneumococcus, and the like.

The new quinolones have a basic ring, a fluorine atom at position 6 of the basic ring and a basic ring group at position 7 of the basic ring. They exhibit a high antibacterial activity against not only Gram-negative bacteria but also Gram-positive bacteria and thus are used for pneumococcus, streptococcus, and the like. The new quinolones are known to exhibit topoisomerase of bacteria.

The DNA topoisomerase of bacteria is an important enzyme for replication of bacteria and is classified into type I topoisomerase and type II topoisomerase.

The type I topoisomerase is an enzyme that cleaves only one of the DNA double strands, and topoisomerase I and topoisomerase III are classified into the type I topoisomerase.

The type II topoisomerase is an enzyme that cleaves both of the DNA double strands, and the DNA gyrase (topoisomerase II) and the topoisomerase IV are classified into the type II topoisomerase.

The DNA gyrase is encoded by gyrA and gyrB, and topoisomerase IV is encoded by grlA and grlB.

The quinolones act on A subunit of the topoisomerase. The quinolone-resistant bacteria have a mutation in at least one of gyrA and grlA encoding the A subunit, so that they acquire resistance to the quinolones.

<Dosage form>

The dosage form of the antibacterial agent for drug-resistant bacteria is not particularly limited and may be appropriately selected depending on the intended purpose. Examples of the dosage form include an oral solid preparation, an oral liquid preparation, an injection and an inhalation powder.

-Oral solid preparation-

The oral solid preparation is not particularly limited and may be appropriately selected depending on the intended purpose. Examples of the oral solid preparation include a tablet, a coated tablet, granules, powder and a capsule.

The method for producing the oral solid preparation is not particularly limited and may be a routine method. For example, the oral solid preparation can be produced by adding an excipient and, if necessary, the above other ingredients and various additives to at least one of the antibacterial agents of General Formula (1) and the derivative thereof. Here, the excipient is not particularly limited and may be appropriately selected depending on the intended purpose. Examples of the excipient include lactose, sucrose, sodium chloride, glucose, starch, calcium carbonate, kaolin, microcrystalline cellulose and silicic acid. The additives are not particularly limited and may be appropriately selected depending on the intended purpose. Examples of the additives include a binding agent, a disintegrating agent, a lubricating agent, a coloring agent and a sweetening/flavoring agent.

The binding agent is not particularly limited and may be appropriately selected depending on the intended purpose. Examples of the binding agent include water, ethanol, propanol, simple syrup, glucose liquid, starch liquid, gelatine liquid, carboxymethylcellulose, hydroxypropylcellulose, hydroxypropylstarch, methylcellulose, ethylcellulose, shellac, calcium phosphate and polyvinylpyrrolidone.

The disintegrating agent is not particularly limited and may be appropriately selected depending on the intended purpose. Examples of the disintegrating agent include dry starch, sodium alginate, powdered agar, sodium hydrogencarbonate, calcium carbonate, sodium lauryl sulfate, monoglyceride stearate and lactose.

The lubricating agent is not particularly limited and may be appropriately selected depending on the intended purpose. Examples of the lubricating agent include purified talc, stearic acid salts, borax and polyethylene glycol.

The coloring agent is not particularly limited and may be appropriately selected depending on the intended purpose. Examples of the coloring agent include titanium oxide and iron oxide.

The sweetening/flavoring agent is not particularly limited and may be appropriately selected depending on the intended purpose. Examples of the sweetening/flavoring agent include sucrose, orange peel, citric acid and tartaric acid.

-Oral liquid preparation-

The oral liquid preparation is not particularly limited and may be appropriately selected depending on the intended purpose. Examples of the oral liquid preparation include an internal liquid, syrup and elixir.

The method for producing the oral liquid preparation is not particularly limited and may be a routine method. For example, the oral liquid preparation can be produced by adding additives to at least one of the antibacterial agent of General Formula (1) and the derivative thereof and optionally used other ingredients described above. Here, the

additives are not particularly limited and may be appropriately selected depending on the intended purpose. Examples of the additives include a sweetening/flavoring agent, a buffer and a stabilizing agent.

The sweetening/flavoring agent is not particularly limited and may be appropriately selected depending on the intended purpose. Examples of the sweetening/flavoring agent include sucrose, orange peel, citric acid and tartaric acid.

The buffer is not particularly limited and may be appropriately selected depending on the intended purpose. Examples of the buffer include sodium citrate.

The stabilizing agent is not particularly limited and may be appropriately selected depending on the intended purpose. Examples of the stabilizing agent include tragacanth, gum arabic and gelatin.

-Injection-

The injection is not particularly limited and may be appropriately selected depending on the intended purpose. Examples of the injection include a solution, a suspension and a solid preparation reconstituted upon use.

The method for producing the injection is not particularly limited and may be a routine method. For example, the injection can be produced by adding a pH adjuster, a buffer, a stabilizing agent, a tonicity agent, a local anesthetic, etc. to at least one of the antibacterial agent of General Formula (1) and the derivative thereof and optionally used other ingredients described above. Here, the pH adjuster or buffer is not particularly limited and may be appropriately selected depending on the intended purpose. Examples of the pH adjuster or buffer include sodium citrate, sodium acetate and sodium phosphate. The stabilizing agent is not particularly limited and may be appropriately selected depending on the intended purpose. Examples of the stabilizing agent include sodium pyrosulfite, EDTA, thioglycolic acid and thiolactic acid. The tonicity agent is not particularly limited and may be appropriately selected depending on the intended purpose. Examples of the tonicity agent include sodium chloride and glucose. The local anesthetic is not particularly limited and may be appropriately selected depending on the intended purpose. Examples of the local anesthetic include procaine hydrochloride and lidocaine hydrochloride.

<Administration>

Regarding the antibacterial agent for use in treating an individual infected with a drug-resistant bacteria according to the present invention, the administration method, the administration dose, the timing of administration and the subject to be administered are not particularly limited and may be appropriately selected depending on the intended purpose.

The administration method is not particularly limited and may be appropriately selected depending on the intended purpose. Examples of the administration method include oral administration, injection and inhalation.

The administration dose is not particularly limited and may be appropriately selected considering various factors of a subject to be administered, such as the age, body weight, constitution, symptom and the presence or absence of administration of a drug containing other active ingredients.

The animal species serving as the subject to be administered is not particularly limited and may be appropriately selected depending on the intended purpose. Examples of the animal species include human, monkey, pig, bovine, sheep, goat, dog, cat, mouse, rat and bird. Among them, the antibacterial agent for use in treating an individual infected with a drug-resistant bacteria is suitably administered to human.

<Antibacterial activity>

When drug-resistant bacteria targeted by the antibacterial agent for use in treating an individual infected with a drug-resistant bacteria of the present invention include at least quinolone-resistant bacteria, the antibacterial agent for drug-resistant bacteria preferably exhibits an antibacterial activity against quinolone-resistant bacteria whose topoisomerase gene has been mutated. Furthermore, the antibacterial agent for drug-resistant bacteria is more preferably ineffective to quinolone-susceptible bacteria.

The method for measuring the antibacterial activity is not particularly limited and may be appropriately selected depending on the intended purpose. Examples thereof include a method in which the minimum inhibitory concentration (hereinafter may be referred to as "MIC") is measured.

The method for measuring the MIC is not particularly limited and may be appropriately selected from known methods.

The MIC of the antibacterial agent for use in treating an individual infected with a drug-resistant bacteria against the drug-resistant bacteria, especially the quinolone-resistant bacteria, is not particularly limited and may be appropriately selected depending on, for example, the type of the bacteria to be targeted. The MIC is preferably lower than 8 mg/L, more preferably 4 mg/L or lower, still more preferably 2 mg/L or lower, particularly preferably 1 mg/L or lower. When the MIC is 8 mg/L or higher, the antibacterial agent for drug-resistant bacteria cannot inhibit the growth of the bacteria in some cases, since the antibacterial activity is low.

Also, in the present invention, the description "the antibacterial agent for use in treating an individual infected with a drug-resistant bacteria of the present invention is ineffective to quinolone-susceptible bacteria" also encompasses the case where the antibacterial activity against the quinolone-susceptible bacteria is low. The MIC of the antibacterial agent for drug-resistant bacteria against the quinolone-susceptible bacteria is not particularly limited and may be appropriately selected depending on the intended purpose depending on, for example, the type of the bacteria. It is preferably 8 mg/L or higher.

<Use>

The antibacterial agent for use in treating an individual infected with a drug-resistant bacteria according to the present invention may be used alone or in combination with a drug containing other active ingredients. Also, the antibacterial agent for drug-resistant bacteria may be formulated into a drug containing other active ingredients before use.
The antibacterial agent for use in treating an individual infected with a drug-resistant bacteria according to the present invention preferably exhibits no antibacterial activity against drug-susceptible bacteria in order to prevent the drug-susceptible bacteria from becoming resistant. Thus, particularly preferably, the antibacterial agent for drug-resistant bacteria is used in combination with or is formulated into a drug containing other active ingredients that exhibits an antibacterial activity against drug-susceptible bacteria only.

<Application>

The antibacterial agent for use in treating an individual infected with a drug-resistant bacteria contains the antibacterial agent of General Formula (1), and exhibits an excellent antibacterial activity against drug-resistant bacteria resistant to at least one drug. Thus, it can suitably be used for an infectious disease therapeutic drug or an infectious disease combined therapeutic drug of the present invention.

(Infectious disease therapeutic drug and infectious disease combined therapeutic drug)

<Infectious disease therapeutic drug>

An infectious disease therapeutic drug for use in treating an individual infected with a drug-resistant bacteria of the present invention contains the antibacterial agent for drug-resistant bacteria of the present invention; and, if necessary, further contains other ingredients.

<<Antibacterial agent for drug-resistant bacteria>>

The amount of the antibacterial agent for use in treating an individual infected with a drug-resistant bacteria according to the present invention contained in the infectious disease therapeutic drug is not particularly limited and may be appropriately selected depending on the intended purpose. Also, the infectious disease therapeutic drug may be the antibacterial agent for drug-resistant bacteria itself.

<<Other ingredients>>

The other ingredients contained in the infectious disease therapeutic drug for use in treating an individual infected with a drug-resistant bacteria according to the present invention are not particularly limited and may be appropriately selected depending on the intended purpose. Examples of the other ingredients include pharmacologically acceptable carriers such as ethanol, water and starch.
The amount of the other ingredients contained in the infectious disease therapeutic drug for use in treating an individual infected with a drug-resistant bacteria according to the present invention is not particularly limited and may be appropriately selected depending on the intended purpose from a range within which the effects of at least one of the antibacterial agent of General Formula (1) and the derivative thereof are not impeded.

<<Use>>

The infectious disease therapeutic drug for use in treating an individual infected with a drug-resistant bacteria according to the present invention may be used alone or in combination with a drug containing other active ingredients. Also, the antibacterial agent for drug-resistant bacteria may be formulated into a drug containing other active ingredients before use.
The infectious disease therapeutic drug for use in treating an individual infected with a drug-resistant bacteria

according to the present invention preferably exhibits no antibacterial activity against drug-susceptible bacteria in order to prevent the drug-susceptible bacteria from becoming resistant. Thus, preferably, the infectious disease therapeutic drug is used in combination with or is formulated into a drug containing other active ingredients that exhibits an antibacterial activity against drug-susceptible bacteria only.

<Infectious disease combined therapeutic drug>

An infectious disease combined therapeutic drug for use in treating an individual infected with a drug-resistant bacteria of the present invention is a therapeutic drug using in combination the antibacterial agent for drug-resistant bacteria of the present invention and at least one quinolone.

<<Antibacterial agent for drug-resistant bacteria>>

The amount of the antibacterial agent for use in treating an individual infected with a drug-resistant bacteria according to the present invention contained in the infectious disease combined therapeutic drug of the present invention is not particularly limited and may be appropriately selected depending on the intended purpose.

<<Quinolone>>

The quinolone used in the infectious disease combined therapeutic drug for use in treating an individual infected with a drug-resistant bacteria according to the present invention is not particularly limited and may be appropriately selected depending on the intended purpose. Examples of the quinolone include the above old quinolones and the above new quinolones. These may be used alone or in combination.

Examples of the old quinolones include nalidixic acid, piromidic acid and pipemidic acid.

Examples of the new quinolones include norfloxacin, levofloxacin, ofloxacin, enoxacin, ciprofloxacin chloride, tosufloxacin tosylate, lomefloxacin hydrochloride, fleroxacin, sparfloxacin, gatifloxacin and pazufloxacin mesylate.

The amount of the quinolone contained in the infectious disease combined therapeutic drug for use in treating an individual infected with a drug-resistant bacteria according to the present invention is not particularly limited and may be appropriately selected depending on, for example, the type of the quinolone.

<<Use in combination>>

The above wording "using in combination" refers to a case where the antibacterial agent for use in treating an individual infected with a drug-resistant bacteria for use in treating an individual infected with a drug-resistant bacteria according to of the present invention and at least one of the quinolones are used in combination and a case where at least one of the quinolones is formulated into the antibacterial agent for drug-resistant bacteria in use.

By using them in combination, the antibacterial agent for use in treating an individual infected with a drug-resistant bacteria of the present invention contained in the infectious disease combined therapeutic drug acts on drug-resistant bacteria, especially quinolone-resistant bacteria, while the quinolone contained in the infectious disease combined therapeutic drug acts on drug-susceptible bacteria, especially quinolone-susceptible bacteria. Thus, it is advantageous in that the infectious disease combined therapeutic drug for use in treating an individual infected with a drug-resistant bacteria according to the present invention can exhibit an antibacterial activity regardless of the drug resistance of the bacteria causing infectious diseases.

Furthermore, the infectious disease combined therapeutic drug of the present invention is advantageous in that the antibacterial agent for drug-resistant bacteria contained therein suppresses appearance of quinolone-resistant bacteria since it is effective to quinolone-resistant bacteria selected by use of quinolones.

<<Other ingredients>>

When the infectious disease combined therapeutic drug is used as a formulation containing the antibacterial agent for use in treating an individual infected with a drug-resistant bacteria according to the present invention and at least one of the quinolones, the formulation may further contain other ingredients, if necessary.

The other ingredients are not particularly limited and may be appropriately selected depending on the intended purpose. Examples of the other ingredients include pharmacologically acceptable carriers such as ethanol, water and starch.

The amount of the other ingredients contained in the infectious disease combined therapeutic drug is not particularly limited and may be appropriately selected depending on the intended purpose from a range within which the effects of at least one of the antibacterial agent of General Formula (1) and the derivative thereof is not impeded.

<Target>

The infectious disease targeted by the infectious disease therapeutic drug or the infectious disease combined therapeutic drug for use in treating an individual infected with a drug-resistant bacteria is not particularly limited and may be appropriately selected depending on the intended purpose. The infectious disease is preferably an infectious disease caused by quinolone-resistant bacteria, particularly preferably at least one of a MRSA infectious disease, a VISA infectious disease and a VRSA infectious disease.

In addition, the infectious disease targeted by the infectious disease therapeutic drug or the infectious disease combined therapeutic drug is preferably an infectious disease caused by Gram-positive bacteria, more preferably an infectious disease caused by enterococcus, still more preferably an infectious disease caused by quinolone-resistant enterococcus.

<Dosage form>

The dosage form of the infectious disease therapeutic drug or the infectious disease combined therapeutic drug for use in treating an individual infected with a drug-resistant bacteria is not particularly limited and may be appropriately selected depending on the intended purpose. Examples of the dosage form include an oral solid preparation, an oral liquid preparation, an injection and an inhalation powder.

-Oral solid preparation-

The oral solid preparation is not particularly limited and may be appropriately selected depending on the intended purpose. Examples of the oral solid preparation include a tablet, a coated tablet, granules, powder and a capsule.

The method for producing the oral solid preparation is not particularly limited and may be a routine method. For example, in the case of the infectious disease therapeutic drug, the oral solid preparation can be produced by adding an excipient and various additives to the antibacterial agent of General Formula (1) for use in treating an individual infected with a drug-resistant bacteria. Meanwhile, in the case of the infectious disease combined therapeutic drug, the oral solid preparation can be produced by adding an excipient and various additives to the antibacterial agent of General Formula (1) for use in treating an individual infected with a drug-resistant bacteria and the quinolone.

Here, the excipient is not particularly limited and may be appropriately selected depending on the intended purpose. Examples of the excipient include lactose, sucrose, sodium chloride, glucose, starch, calcium carbonate, kaolin, microcrystalline cellulose and silicic acid. The additive is not particularly limited and may be appropriately selected depending on the intended purpose. Examples of the additive include a binding agent, a disintegrating agent, a lubricating agent, a coloring agent and a sweetening/flavoring agent.

The binding agent is not particularly limited and may be appropriately selected depending on the intended purpose. Examples of the binding agent include water, ethanol, propanol, simple syrup, glucose liquid, starch liquid, gelatine liquid, carboxymethylcellulose, hydroxypropylcellulose, hydroxypropylstarch, methylcellulose, ethylcellulose, shellac, calcium phosphate and polyvinylpyrrolidone.

The disintegrating agent is not particularly limited and may be appropriately selected depending on the intended purpose. Examples of the disintegrating agent include dry starch, sodium alginate, powdered agar, sodium hydrogencarbonate, calcium carbnate, sodium lauryl sulfate, monoglyceride stearate and lactose.

The lubricating agent is not particularly limited and may be appropriately selected depending on the intended purpose. Examples of the lubricating agent include purified talc, stearic acid salts, borax and polyethylene glycol.

The coloring agent is not particularly limited and may be appropriately selected depending on the intended purpose. Examples of the coloring agent include titanium oxide and iron oxide.

The sweetening/flavoring agent is not particularly limited and may be appropriately selected depending on the intended purpose. Examples of the sweetening/flavoring agent include sucrose, orange peel, citric acid and tartaric acid.

-Oral liquid preparation-

The oral liquid preparation is not particularly limited and may be appropriately selected depending on the intended purpose. Examples of the oral liquid preparation include an internal liquid, syrup and elixir.

The method for producing the oral liquid preparation is not particularly limited and may be a routine method. For example, in the case of the infectious disease therapeutic drug for use in treating an individual infected with a drug-resistant bacteria according to the present invention, the oral liquid preparation can be produced by adding additives to the antibacterial agent for drug-resistant bacteria. Meanwhile, in the case of the infectious disease combined therapeutic drug for use in treating an individual infected with a drug-resistant bacteria according to the present invention, the oral liquid preparation can be produced by adding additives to the antibacterial agent for drug-resistant bacteria and the

quinolone.

Examples of the additive include a sweetening/flavoring agent, a buffer and a stabilizing agent.

The sweetening/flavoring agent is not particularly limited and may be appropriately selected depending on the intended purpose. Examples of the sweetening/flavoring agent include sucrose, orange peel, citric acid and tartaric acid.

The buffer is not particularly limited and may be appropriately selected depending on the intended purpose. Examples of the buffer include sodium citrate.

The stabilizing agent is not particularly limited and may be appropriately selected depending on the intended purpose. Examples of the stabilizing agent include tragacanth, gum arabic and gelatin.

-Injection-

The injection is not particularly limited and may be appropriately selected depending on the intended purpose. Examples of the injection include a solution, a suspension and a solid preparation reconstituted upon use.

The method for producing the injection is not particularly limited and may be a routine method. For example, in the case of the infectious disease therapeutic drug of the present invention, the injection can be produced by adding a pH adjuster, a buffer, a stabilizing agent, a tonicity agent, a local anesthetic, etc. to the antibacterial agent for drug-resistant bacteria. Meanwhile, in the case of the infectious disease combined therapeutic drug of the present invention, the injection can be produced by adding a pH adjuster, a buffer, a stabilizing agent, a tonicity agent, a local anesthetic, etc. to the antibacterial agent for drug-resistant bacteria and the quinolone.

Here, the pH adjuster or buffer is not particularly limited and may be appropriately selected depending on the intended purpose. Examples of the pH adjuster or buffer include sodium citrate, sodium acetate and sodium phosphate. The stabilizing agent is not particularly limited and may be appropriately selected depending on the intended purpose. Examples of the stabilizing agent include sodium pyrosulfite, EDTA, thioglycolic acid and thiolactic acid. The tonicity agent is not particularly limited and may be appropriately selected depending on the intended purpose. Examples of the tonicity agent include sodium chloride and glucose. The local anesthetic is not particularly limited and may be appropriately selected depending on the intended purpose. Examples of the local anesthetic include procaine hydrochloride and lidocaine hydrochloride.

<Administration>

In the infectious disease therapeutic drug or infectious disease combined therapeutic drug for use in treating an individual infected with a drug-resistant bacteria according to the present invention, the administration method, the administration dose, the time of administration and the subject to be administered are not particularly limited and may be appropriately selected depending on the intended purpose.

The administration method is not particularly limited and may be appropriately selected depending on the intended purpose. Examples of the administration method include oral administration, injection and inhalation.

Notably, in the infectious disease combined therapeutic drug according to the present invention, the antibacterial agent for drug-resistant bacteria according to the present invention and the quinolone may be administered simultaneously or separately.

The administration dose is not particularly limited and may be appropriately selected considering variour factors of a subject to be administered, such as the age, body weight, constitution, symptom and the presence or absence of administration of a drug containing other active ingredients.

The animal species serving as the subject to be administered is not particularly limited and may be appropriately selected depending on the intended purpose. Examples of the animal species include human, monkey, pig, bovine, sheep, goat, dog, cat, mouse, rat and bird. Among them, the antibacterial agent for drug-resistant bacteria is suitably administered to human.

<Application>

Since the infectious disease therapeutic drug and the infectious disease combined therapeutic drug for use in treating an individual infected with a drug-resistant bacteria according to the present invention each contain the antibacterial agent for drug-resistant bacteria of the present invention, they exhibit an excellent antibacterial activity against drug-resistant bacteria without permitting drug-susceptible bacteria to become resistant. Thus, the infectious disease therapeutic drug and the infectious disease combined therapeutic drug can suitably be used for an infectious disease caused by quinolone-resistant bacteria, especially at least one of a MRSA infectious disease, a VISA infectious disease and a VRSA infectious disease. Also, the infectious disease therapeutic drug and the infectious disease combined therapeutic drug can suitably be used for an infectious disease caused by enterococcus, especially an infectious disease caused by quinolone-resistant enterococcus.

Conventionally, whether a quinolone-containing drug is administered to a patient has been determined by a susceptibility test on infection-causing bacteria separated from the patient to examine whether the infectious disease the patient suffers from is caused by quinolone-susceptible bacteria or quinolone-resistant bacteria. It takes two to three days for this susceptibility test to give examination results, and as a result the initiation of treatment is delayed problematically. In contrast, the infectious disease combined therapeutic drug of the present invention is advantageous in that it can initiate treatment early without requiring such a susceptibility test.

(Screening method)

A non-claimed screening method for an antibacterial agent for drug-resistant bacteria includes separately culturing quinolone-susceptible bacteria and quinolone-resistant bacteria in the presence of a test sample at the same concentration, and selecting the test sample that does not suppress the growth of the quinolone-susceptible bacteria and suppresses the growth of the quinolone-resistant bacteria.

<Quinolone-susceptible bacteria and quinolone-resistant bacteria>

The type of bacteria used as the quinolone-susceptible bacteria and the quinolone-resistant bacteria is not particularly limited and may be appropriately selected depending on the intended purpose. Examples of the bacteria include pathogenic staphylococci (e.g., Staphylococcus aureus and Staphylococcus epidermidis), nonpathogenic staphylococci other than the above pathogenic staphylococci, and enterococci.

The quinolone-susceptible Staphylococcus aureus is not particularly limited and may be appropriately selected depending on the intended purpose. Examples thereof include standard strains (e.g., FDA209P) and quinolone-susceptible Staphylococcus aureus isolates obtained from healthy humans and patients.

The quinolone-resistant Staphylococcus aureus is not particularly limited and may be appropriately selected depending on the intended purpose. Examples thereof include VRSA typified by VRS1 (see Périchon B, Courvalin P., Antimicrob Agents Chemother, 2009, Nov; 53(11), 4580-7, Epub 2009 Jun 8), community-acquired multi-resistant MRSA (CA-MRSA) typified by USA300FPR3757 (see Diep BA, Gill SR, Chang RF, Phan TH, Chen JH, Davidson MG, Lin F, Lin J, Carleton HA, Mongodin EF, Sensabaugh GF, "Perdreau-Remington F. Complete genome sequence of USA300, an epidemic clone of community-acquired meticillin-resistant Staphylococcus aureus." Lancet., 2006, Mar 4;367(9512), 731-9.), and quinolone-resistant isolates such as VISA typified by Mu50 (see Frontiers in Antimicrobial Resistance. Edited by White, D.G., Alekshun, M.N. and McDermott, P.F. 2005, ASM Press American Society for Microbiology Washington, DC.). Notably, as shown in Table 2-2 in the below-described Examples, the USA300FPR3757 is a bacterial strain having resistance to quinolones.

Prior to screening, preferably, the quinolone-susceptible bacteria and the quinolone-resistant bacteria are precultured so that the number of bacteria is adjusted to a desired number.

The medium for preparing the quinolone-susceptible bacteria and the quinolone-resistant bacteria is not particularly limited and may be appropriately selected depending on the intended purpose. Examples thereof include media such as BHI (Brain Heart Infusion) and MH (Mueller Hinton) and physiological saline.

The amount of the quinolone-susceptible bacteria or the quinolone-resistant bacteria added is not particularly limited and may be appropriately selected depending on the intended purpose. The amount thereof is preferably $1 \times 10^3$ CFU/mL to $1 \times 10^5$ CFU/mL, more preferably $1 \times 10^4$ CFU/mL. When the amount thereof is less than $1 \times 10^3$ CFU/mL, the bacteria insufficiently grow even in the presence of a test sample having no antibacterial activity, resulting in that the antibacterial activity cannot accurately be evaluated in some cases. Whereas when the amount thereof is more than $1 \times 10^5$ CFU/mL, a test sample having an antibacterial activity cannot accurately be evaluated for antibacterial activity in some cases, since the number of the bacteria is too great.

The method for adjusting the number of the quinolone-susceptible bacteria or the quinolone-resistant bacteria added is not particularly limited and may be appropriately selected depending on the intended purpose. Examples of the method include a method in which the number of the bacteria is adjusted based on turbidity measured by an absorption spectrometer.

The culture temperature, culture period and culture method are not particularly limited and may be appropriately selected depending on, for example, the type of the bacteria.

When the screening is performed using the quinolone-susceptible Staphylococcus aureus and the quinolone-resistant Staphylococcus aureus, the culture temperature is preferably 33°C to 38°C, more preferably 35°C to 37°C.

When the screening is performed using the quinolone-susceptible Staphylococcus aureus and the quinolone-resistant Staphylococcus aureus, the culture period is preferably 16 hours to 48 hours, more preferably 16 hours to 20 hours.

When the screening is performed using the quinolone-susceptible Staphylococcus aureus and the quinolone-resistant Staphylococcus aureus, the culture method is not particularly limited and may be appropriately selected depending on the intended purpose. Examples thereof include static culturing and shake culturing.

<Test sample>

The test sample is not particularly limited and may be appropriately selected depending on the intended purpose. Examples of the test sample include culture supernatants obtained by culturing microorganisms, compounds, DNA, proteins, extracts from animals and plants, synthetic sugar chains, and peptides.

The type or amount of the solvent for suspending the test sample is not particularly limited, as long as it does not suppress the growth of bacteria, and may be appropriately selected depending on the intended purpose.

The concentration of the test sample added is not particularly limited, as long as all of the test samples are added at the same concentration, and may be appropriately selected depending on, for example, the type of the test sample.

The concentration of a substance having an antibacterial activity contained in the test sample is unknown. Thus, when the concentration of the substance having an antibacterial activity contained in the test sample is too high, the growth of both the quinolone-susceptible bacteria and the quinolone-resistant bacteria is suppressed, resulting in failure to select the substance of interest. In this case, after all of the test samples have been further diluted to the same concentration, screening may be performed again.

<Selection>

The selection method is not particularly limited, as long as it can determine whether the quinolone-susceptible bacteria and the quinolone-resistant bacteria grow or not, and may be appropriately selected depending on the intended purpose. Examples of the selection method include a method in which the presence or absence of the growth of bacteria is visually determined based on turbidity and a method in which the turbidity is measured based on absorbance. Among them, preferred is a method in which the growth of bacteria is visually determined, since it can surely determine whether the bacteria grow or not.

In the above selection, preferably, the concentration of the test sample is set to one concentration for a primary screening in terms of screening efficiency. If necessary, the test sample may be further diluted serially.

Whether the quinolone-susceptible bacteria grow or not can be determined by comparing the growth of the quinolone-susceptible bacteria in the absence of the test sample (hereinafter may be referred to as "quinolone-susceptible bacteria control") with the growth of the quinolone-susceptible bacteria in the presence of the test sample.

Similarly, whether the quinolone-resistant bacteria grow or not can be determined by comparing the growth of the quinolone-resistant bacteria in the absence of the test sample (hereinafter may be referred to as "quinolone-resistant bacteria control") with the growth of the quinolone-resistant bacteria in the presence of the test sample.

Here, the suppressive rate of the test sample against the growth of the quinolone-susceptible bacteria (hereinafter may be referred to as "quinolone-susceptible bacteria growth suppressive rate") and the suppressive rate of the test sample against the growth of the quinolone-resistant bacteria (hereinafter may be referred to as "quinolone-resistant bacteria growth suppressive rate") can be calculated by the following formulas.

Quinolone-susceptible bacteria growth suppressive rate (%) = (the turbidity of quinolone-susceptible bacteria control − the turbidity of quinolone-susceptible bacteria in the presence of the test sample)/the turbidity of quinolone-susceptible bacteria control × 100

Quinolone-resistant bacteria growth suppressive rate (%) = (the turbidity of quinolone-resistant bacteria control − the turbidity of quinolone-resistant bacteria in the presence of the test sample)/the turbidity of quinolone-resistant bacteria control × 100

Here, the criteria for determining whether or not the test sample suppresses the growth of the quinolone-susceptible bacteria or the quinolone-resistant bacteria are not particularly limited and may be appropriately selected depending on the intended purpose. In either case, the above-described suppressive rate is preferably 80% or higher, more preferably 95% or higher, still more preferably 99% or higher.

The criteria for selecting the test sample that does not suppress the growth of the quinolone-susceptible bacteria and suppresses the growth of the quinolone-resistant bacteria are not particularly limited and may be appropriately selected depending on the intended purpose, as long as the quinolone-resistant bacteria growth suppressive rate is higher than the quinolone-susceptible bacteria growth suppressive rate. The quinolone-resistant bacteria growth suppressive rate is preferably 2 or more times, more preferably 4 or more times, still more preferably 8 or more times, particularly preferably 16 or more times the quinolone-susceptible bacteria growth suppressive rate (i.e., the quinolone-resistant bacteria growth suppressive rate/quinolone-susceptible bacteria growth suppressive rate).

Alternatively, the selection may be performed as follows. Specifically, the quinolone-susceptible bacteria and the quinolone-resistant bacteria are cultured in the test sample which has serially been 2-fold diluted, and the titer of the test sample is obtained from the maximum dilution degree at which the growth of them is suppressed.

The above titer can be obtained as a ratio of the maximum dilution degree at which the growth of the quinolone-resistant bacteria is suppressed to the maximum dilution degree at which the growth of the quinolone-susceptible bacteria is suppressed (i.e., the maximum dilution degree at which the growth of the quinolone-resistant bacteria is suppressed/the maximum dilution degree at which the growth of the quinolone-susceptible bacteria is suppressed).

The selection based on the titer may be performed as the secondary screening after the primary screening; or may be performed on the test sample in which no difference in growth between the quinolone-susceptible bacteria and the quinolone-resistant bacteria has been observed at the primary screening.

The titer is not particularly limited and may be appropriately selected depending on the intended purpose. The ratio of the maximum dilution degree at which the growth of the quinolone-resistant bacteria is suppressed to the maximum dilution degree at which the growth of the quinolone-susceptible bacteria is suppressed is preferably 2 or more, more preferably 4 or more, still more preferably 8 or more, particularly preferably 16 or more.

The test sample selected in this manner acts on the quinolone-resistant bacteria only to suppress their growth without acting on the quinolone-susceptible bacteria. Thus, it does not permit the quinolone-susceptible bacteria to be resistant while exhibits an excellent antibacterial activity against the quinolone-resistant bacteria. Therefore, the selected test sample can be suitably used for the antibacterial agent for drug-resistant bacteria of the present invention.

Notably, when the antibacterial agent for drug-resistant bacteria selected by the above-described screening method is used as an antibacterial agent for quinolone-resistant bacteria, the test sample that acts strongly on at least one of the mutated topoisomerase IV and the mutated DNA gyrase may further be selected after the above screening by confirming its affinity to at least one of the mutated topoisomerase IV and the mutated DNA gyrase or at least one of the native topoisomerase IV and the native DNA gyrase.

In one employable method for confirming the affinity to at least one of the mutated topoisomerase IV and the mutated DNA gyrase, the MIC of the test sample is measured using a set of bacterial strains sharing the same genetic background, where one of them has native genes of topoisomerase IV and DNA gyrase and the other has a mutated gene of at least one of topoisomerase IV and DNA gyrase. Here, the lower the MIC value for the bacterial strain having a mutated gene of at least one of topoisomerase IV and DNA gyrase than the MIC value for the bacterial strain having the native genes, the more suitably the test sample can be used as an antibacterial agent for quinolone-resistant bacteria.

## Examples

The present invention will next be described in detail by way of Examples of the present invention. The present invention should not be construed as being limited to the Examples. In the following Examples and Test Examples, the unit "%" means "% by mass" unless otherwise specified.

### (Example 1: Screening for substance effective to drug-resistant bacteria)

<Screening sample>

The screening samples used were 1,928 types of culture supernatants obtained by separating from soil 1,928 types of actinomycetes including the below-described Streptomyces hyalinum-MB891-A1 strain (ATCC29817) and culturing them by a known culture method.

-Culture of Streptomyces hyalinum-

The culturing method for Streptomyces hyalinum will be described below as one exemplary culturing method of actinomycetes.

Microorganisms of Streptomyces hyalinum-MB891-A1 strain (ATCC29817) were slant-cultured on an agar medium. Separately, a liquid medium (whose pH was adjusted to 7.0) containing galactose 2%, dextrin 2%, bactosoytone (product of Difco K.K.) 1%, corn steep liquor 0.5%, ammonium sulfate 0.2% and calcium carbonate 0.2% was dispensed into conical flasks (volume: 500 mL) so that each conical flask contained 110 mL of the liquid medium. Then the conical flasks were sterilized at 120°C for 20 min by a routine method. The above-cultured microorganisms were inoculated into each conical flask, followed by shake-culturing under rotation at 30°C for 2 days, to thereby obtain a seed culture liquid.
A liquid medium (whose pH was adjusted to 7.0) containing corn steep liquor 0.5%, glycerin 0.5%, sucrose 2.0%, dried yeast (product of Oriental Yeast Co., Ltd.) 1.0%, soybean meal (product of Nisshin Seiyu K.K.) 1.0% and cobalt chloride hexahydrate 0.00001% was dispensed into conical flasks (volume: 500 mL) so that each conical flask contained 110 mL of the liquid medium. The conical flasks were sterilized at 120°C for 20 min by a routine method to prepare production media. Then, 2% by volume of the seed culture liquid was inoculated into each production medium, followed by shake-culturing under rotation at 27°C for 5 days (180 rpm).
The culture liquid (110 mL) obtained in this manner was centrifuged to obtain a culture filtrate. The obtained culture filtrate was dispensed into the wells of a 96-well plate at 100 μL/well, and concentrated and dried under reduced pressure. Then, 70% methanol was added to the wells at 100 μL/well, followed by being left to stand on ice for 3 hours to 5 hours for dissolution.

<Screening>

-Culturing of drug-susceptible Staphylococcus aureus and drug-resistant

Staphylococcus aureus-

The drug-susceptible Staphylococcus aureus used was FDA209P (ATCC6538P). Also, the drug-resistant Staphylococcus aureus used was Mu50 (see Hiramatsu, K., Hanaki, H., et al., J Antimicrob Chemother, 1997, 40(1), 135-6.) which is a typical VISA.
FDA209P and Mu50 were each shake-cultured at 37°C overnight in a BHI medium (product of Becton-Dickinson) (4 mL).
After completion of culturing, the culture liquid of Mu50 or FDA209P was adjusted in absorbance with the BHI medium so that the absorbance at 578 nm of the culture liquid was adjusted to 0.3 measured by an absorption spectrometer (product of Biochrom). In addition, the resultant culture liquid was 1,000-fold diluted with the BHI medium. Each of the thus-prepared bacterial cultures was found to show about $1 \times 10^5$ CFU/mL.

-Screening-

The bacterial cultures of Mu50 and FDA209P were added to the wells of a Round Bottom Cell Culture Plate (product of Corning Inc., #3799, 96 wells) at 100 μL/well (about $1 \times 10^4$ CFU/well). The screening sample, which had been suspended in 70% methanol, was added to the wells at 2.5 μL/well (40-fold dilution), followed by statically culturing at 37°C overnight. As a control, a well containing no screening sample was provided. Whether the growth of Mu50 or FDA209P was suppressed by the screening sample was determined through comparison with the control.
After completion of culturing, the growth of the bacteria was visually determined based on turbidity. The sample that suppressed the growth of Mu50 to four or more times greater extent as compared with the suppression of the growth of FDA209P was selected as a substance exhibiting an antibacterial activity against drug-resistant Staphylococcus aureus. Here, the number of the selected samples was one sample out of the 1,928 screening samples.

(Test Example 1: Measurement of titer of substance exhibiting antibacterial activity)

The sample selected in Example 1 was measured for titer.
Mu50 and FDA209P were cultured in the same manner as in Example 1. After the culture liquids had been adjusted in absorbance with the BHI medium so that the absorbance at 578 nm of each culture liquid was adjusted to 0.3 measured by an absorption spectrometer, the resultant culture liquids were 500-fold diluted with the BHI medium. Each of the thus-prepared bacterial cultures was found to show about $2 \times 10^5$ CFU/mL.
The BHI medium and the sample selected in Example 1 were added to a titration plate (product of Corning Inc.) at 100 μL/well and 2.5 μL/well (40-fold dilution), respectively. From this state, 2-fold serial dilution was performed seven times so as to attain 5,120-fold dilution.
The 500-fold diluted Mu50 and FDA209P bacterial cultures were added at 50 μL/well to the BHI medium containing the sample at various concentrations (50 μL/well), followed by statically culturing at 37°C overnight.
After completion of culturing, the presence or absence of the growth of each strain in each well was visually

determined based on turbidity to calculate the ratio of the titer for Mu50 to the titer for FDA209P (Mu50/FDA209P). When the titer for Mu50 was four or more times greater than the titer for FDA209P, the sample was considered as having an antibacterial activity.

As a result, the titer of the culture supernatant of Streptomyces hyalinum was found to be lower than 40 for FDA209P and to be 160 for Mu50. That is, it was found that the titer for Mu50 was four or more times greater than the titer for FDA209P.

(Test Example 2: Synthesis of culture supernatant of Streptomyces hyalinum)

As shown from the results obtained in Test Example 1, the culture supernatant of Streptomyces hyalinuum exhibited a low growth suppressive activity against FDA209P and a high growth suppressive activity against Mu50. Thus, the substances contained in the culture supernatant of Streptomyces hyalinuum were analyzed in more detail.

<Culture>

Streptomyces hyalinuum was cultured in the same manner as in Example 1 to obtain a culture filtrate. The culture filtrate was extracted with 3 L of butanol to obtain a butanol extract. The butanol was evaporated from the butanol extract under reduced pressure to obtain 1.2 g of an extract. Subsequently, 500 mL of hexane was added to the extract, followed by thoroughly stirring. The resultant mixture was fractionated with a centrifuge into a hexane-soluble fraction and a hexane-insoluble fraction. Then, chloroform (300 mL), methanol (360 mL) and water (240 mL) were added to the hexane-insoluble fraction, and the resultant mixture was thoroughly stirred for partition. The lower layer was separated therefrom, followed by concentrating and drying under reduced pressure, to thereby obtain 182 mg of a crude extract.

The crude extract was subjected to chromatography using a centrifugal liquid-liquid partition chromatograph (product of Sanki Engineering K.K.). In the centrifugal liquid-liquid partition chromatograph, the stationary layer (250 mL, 600 rpm) used was the lower layer of a solvent partitioned at a solvent ratio of chloroform : methanol : water = 5 : 6 : 4 (by volume). The mobile layer used was the upper layer of a solvent partitioned at a solvent ratio of chloroform : methanol : water = 5 : 6 : 4 (by volume). The mobile layer was allowed to flow at a flow rate of 8 mL/min. Then, 95 min after, the mobile layer was changed to the lower layer of a solvent partitioned at a solvent ratio of chloroform : methanol : water = 5 : 6 : 4 (by volume) and was allowed to flow at a flow rate of 8 mL/min to perform chromatography for 21 min. Here, 12 mL was fractionated for each fraction.

Each of the fractions obtained through the above chromatography was concentrated/dried and then dissolved in 100 μL of methanol.

[HPLC conditions]

Solvent: 0.01% trifluoroacetic acid-containing acetonitrile-0.01% trifluoroacetic acid-containing water (two-liquid gradient) (5% (0 min)-100% (30 min)-100% (15 min); 0.01% trifluoroacetic acid-containing acetonitrile) 45 min in total
Column: Shiseido capcell pak UG120 4.6 mm × 150 mm
Column temp.: 25°C
Flow rate: 1.0 mL/min
Detector: PDA
Fraction: 90 fractions

<Measurement of antibacterial activity>

Each of Mu50 and FDA209P was cultured in the same manner as in Example 1, and then the resultant cultures were diluted in the same manner as in Example 1 (each of the prepared bacterial cultures was found to show about 1 × $10^5$ CFU/mL).

The bacterial cultures of Mu50 and FDA209P were added to the wells of a Round Bottom Cell Culture Plate (product of Corning Inc., #3799, 96 wells) at 100 μL/well (about 1 × $10^4$ CFU/well). The above-obtained HPLC fraction, which had been suspended in 70% methanol, was added to the wells at 5 μL/well (20-fold dilution), followed by shake-culturing at 37°C overnight. As a control, a well containing no HPLC fraction was provided.

After completion of culturing, the growth of the bacteria was visually determined based on turbidity. The sample that suppressed the growth of Mu50 to four or more times greater extent as compared with the suppression of the growth of FDA209P was selected as a substance exhibiting an antibacterial activity against drug-resistant Staphylococcus aureus. As a result, fraction Nos. 37, 38 and 41 obtained through the above chromatography were found to exhibit an antibacterial activity.

Each of the fraction Nos. 37, 38 and 41 was collected and then concentrated/dried under reduced pressure for

indentification. As a result, each of the fraction Nos. 37 and 38 was found to contain nybomycin as an active ingredient, and the fraction No. 41 was found to contain deoxynybomycin as an active ingredient. The physico-chemical properties of nybomycin and deoxynybomycin are shown below.

Notably, through concentrating/drying of the fractions obtained by the above chromatography, 9.0 mg of pure nybomycin and 9.0 mg of pure deoxynybomycin could be obtained.

[Physico-chemical properties of nybomycin]

(1) Appearance: colorless powder
(2) Molecular formula: $C_{16}H_{14}N_2O_4$
(3) Through high resolution mass spectrometry (HRESIMS: positive-ion mode), the experimental value: m/z 299.102 $(M+H)^+$ and the calculated value: m/z 299.1029 (as $C_{16}H_{15}N_2O_4$)
(4) The chart of the ultraviolet ray absorption spectrum measured in methanol is shown in Fig. 1.
(5) The chart of $^1$H-NMR spectrum (proton nuclear magnetic resonance spectrum) measured at 25°C and 600 MHz in deuterated tirufluoroacetic acid is shown in Fig. 2. Notably, in Fig. 2, "imp" indicates a peak of impurities. Here, the peak is thought to be attributed to the solvent.
(6) The chart of $^{13}$C-NMR spectrum ($^{13}$C nuclear magnetic resonance spectrum) measured at 25°C and 150 MHz in deuterated tirufluoroacetic acid is shown in Fig. 3.

[Physico-chemical properties of deoxynybomycin]

(1) Appearance: colorless powder
(2) Molecular formula: $C_{16}H_{14}N_2O_3$
(3) Through high resolution mass spectrometry (HRESIMS: positive-ion mode), the experimental value: m/z 283.1075 $(M+H)^+$ and the calculated value: m/z 283.1077 (as $C_{16}H_{15}N_2O_3$)
(4) The chart of the ultraviolet ray absorption spectrum measured in methanol is shown in Fig. 4.
(5) The chart of $^1$H-NMR spectrum (proton nuclear magnetic resonance spectrum) measured at 25°C and 600 MHz in deuterated tirufluoroacetic acid is shown in Fig. 5. Notably, in Fig. 5, "imp" indicates a peak of impurities. Here, the peak is thought to be attributed to the solvent.
(6) The chart of $^{13}$C-NMR spectrum ($^{13}$C nuclear magnetic resonance spectrum) measured at 25°C and 150 MHz in deuterated tirufluoroacetic acid is shown in Fig. 6.

(Test Example 3: Confirmation of point of action of nybomycin)

Using the below-described MIC measurement method, the quinolone, nybomycin (hereinafter may be referred to as "NM") and deoxynybomycin (hereinafter may be referred to as "DNM"), which had been purified in Test Example 2, were evaluated for antibacterial activity against each of the bacterial strains shown in Table 1-1; i.e., methicillin-susceptible S.aureus (MSSA), MRSA and mutants thereof.

The quinolones used were norfloxacin (hereinafter may be referred to as "NFLX"), levofloxacin (hereinafter may be referred to as "LVFX"), ofloxacin (hereinafter may be referred to as "OFLX"), ciprofloxacin chloride (hereinafter may be referred to as "CPFX"), tosufloxacin tosylate (hereinafter may be referred to as "TFLX") and sparfloxacin (hereinafter may be referred to as "SPFX").

Here, the MSSA means bacterial strains showing a MIC of lower than 4 mg/L against oxacillin (hereinafter may be referred to as "OXA") which is classified into β-lactams.

Notably, the mutants listed in Table 1-1 were bacterial strains obtained by selecting their parent strains with quinolones having sequentially higher activities. Specifically, in A subunit (GyrA) encoded by gyrA of DNA gyrase (topoisomerase II), gyrA was mutated to alter the amino acid thereof; or in topoisomerase IV (GrlA) encoded by grlA, grlA was mutated to alter the amino acid thereof (see Fukuda, H., Hori, S., et al., "Antibacterial activity of gatifloxacin (AM-1155, CG5501, BMS-206584), a newly developed fluoroquinolone, against sequentially acquired quinolone-resistant mutants and the norA transformant of Staphylococcus aureus.", Antimicrob Agents Chemother, 1998, 42(8), 1917-22.). That is, the first-step quinolone-resistant mutants are bacterial strains which are the same as their parent strains (clinical isolates) except that the genes shown in the corresponding parts of Table 1-1 are mutated. The second-step quinolone-resistant mutants are bacterial strains which are the same as the first-step quinolone-resistant mutants except that the genes shown in the corresponding parts of Table 1-1 are further mutated. The third-step quinolone-resistant mutants are

bacterial strains which are the same as the second-step quinolone-resistant mutants except that the genes shown in the corresponding parts of Table 1-1 are further mutated. The fourth-step quinolone-resistant strain is a bacterial strain which is the same as the third-step quinolone-resistant mutant bacteria except that the gene shown in the corresponding part of Table 1-1 is further mutated.

<Measurement of MIC>

-Pre-culture of each bacterial strain-

Each of the bacterial strains listed in Table 1-1 was shake-cultured at 37°C overnight in 4 mL of a medium (Tryptical Soy Broth: TSB, product of Becton Dickinson and Company). FDA209P was used as a control.

After completion of culturing, the resultant culture was suspended in a cation-adjusted Mueller Hinton Broth (CAMHB, product of Becton Dickinson and Company). Then, the obtained bacterial culture was adjusted in absorbance with the CAMHB so that the absorbance at 578 nm of the bacterial culture was adjusted to 0.3 measured by an absorption spectrometer. In addition, the resultant bacterial culture was 500-fold diluted with the CAMHB.

-Preparation of quinolone and nybomycin-

Each of the quinolones and the nybomycins (NM and DNM) was added to CAMHB so as to have a concentration of 256 mg/L. From this state, 2-fold serial dilution was performed 11 times up to 0.125 mg/L.

-Measurement of MIC-

Each of the 500-fold diluted, pre-cultured bacterial cultures was added at 50 µL/well to the CAMHB containing the quinolones and the nybomycins at various concentrations (50 µL/well), followed by statically culturing at 37°C overnight.

After completion of culturing, the growth of the bacteria was visually determined based on turbidity to measure MIC in each bacterial strain. Here, the quinolone-resistant Staphylococcus aureus is a bacterial strain showing a MIC of 4 mg/L or lower against LVFX. Regarding the evaluation criteria of the nybomycins, when the MIC was 4 mg/L or lower, the nybomycins were considered as having an antibacterial activity (being effective). The results are shown in Table 1-2.

Notably, in Test Example 3, whether the nybomycin acted on at least one of the mutated gyrA and the mutated grlA could be determined on the basis of whether the MIC values for nybomycin-susceptible bacteria and nybomycin-resistant bacteria evaluated according to the above evaluation criteria were changed by the mutation of at least one gene of gyrA and grlA. The case where these MIC values were mutually different four or more times was evaluated as "significant." Specifically, as a result of comparison between the MIC value of the nybomycin for the mutant (in which at least one of gyrA and grlA was mutated) and the MIC value of the nybomycin for its parent strain, the case where the ratio of the MIC value against the mutant to the MIC value for its parent strain was 1/4 (mutant/parent strain) was evaluated as "significant."

Table 1-1

| Parent Strain (category) and Mutant | | Mutated gene | Substitution of amino acid by mutation of gene |
|---|---|---|---|
| MS5935 (MSSA) | Clinical isolate MSSA strain (parent strain) | - | None |
| MS5935-1 | First-step quinolone-resistnat mutant | *grlA* | S80F |
| MS5935-2 | Second-step quinolone-reistant mutant | *gyrA* | S84L |
| MS5935-3 | Third-step quinolone-reistant mutant | *grlA* | E84K |
| MS5952 (MSSA) | Clinical isolate MSSA strain (parent strain) | - | None |

(continued)

| Parent Strain (category) and Mutant | | Mutated gene | Substitution of amino acid by mutation of gene |
|---|---|---|---|
| MS5952-1 | First-step quinolone-resistnat mutant | *grlA* | S80Y |
| MS5952-2 | Second-step quinolone-reistant mutant | *gyrA* | S84L |
| MS5952-3 | Third-step quinolone-reistant mutant | *grlA* | A116V |
| MR5867 (MRSA) | Clinical isolate MSSA strain (parent strain) | - | None |
| MR5867-1 | First-step quinolone-resistnat mutant | *grlA* | E84K |
| MR5867-2 | Second-step quinolone-reistant mutant | *gyrA* | S84L |
| MR5867-3 | Third-step quinolone-reistant mutant | *grlA* | S80F |
| MR5867-4 | Fourth-step quinolone-reistant mutant | *gyrA* | E88K |
| FDA209P (MSSA) | MSSA Control | - | None |

Table 1-2

| Parent Strain (category) and Mutant | MIC (mg/L) | | | | | | | |
|---|---|---|---|---|---|---|---|---|
| | Quinolones | | | | | | Nibomycins | |
| | NFLX | LVFX | OFLX | CPFX | TFLX | SPFX | NM | DNM |
| MS5935 (MSSA) | 1 | 0.125 | 0.25 | 0.25 | 0.125 | ≤0.063 | >64 | 32 |
| MS5935-1 | 16 | 0.5 | 1 | 2 | 1 | ≤0.063 | >64 | 16 |
| MS5935-2 | 128 | 8 | 16 | 32 | 64 | 16 | 1 | 0.5 |
| MS5935-3 | 128 | 64 | 128 | 128 | >128 | 32 | 1 | 0.5 |
| MS5952 (MSSA) | 1 | 0.25 | 0.25 | 0.5 | 0.5 | ≤0.063 | >64 | 16 |
| MS5952-1 | 16 | 0.5 | 1 | 2 | 2 | 0.125 | >64 | 8 |
| MS5952-2 | 16 | 4 | 8 | 8 | 8 | 8 | 0.5 | 0.5 |
| MS5952-3 | 128 | 32 | 64 | 64 | >128 | 16 | 0.5 | ≤0.063 |
| MR5867(MRSA) | 1 | 0.125 | 0.25 | 0.25 | ≤0.063 | ≤0.063 | >64 | 8 |
| MR5867-1 | 16 | 0.5 | 1 | 2 | 0.25 | ≤0.063 | >64 | 8 |
| MR5867-2 | 64 | 8 | 16 | 32 | 8 | 8 | 0.25 | ≤0.063 |
| MR5867-3 | >128 | 16 | 64 | 128 | >128 | 16 | 0.25 | ≤0.063 |
| MR5867-4 | >128 | >128 | >128 | 128 | >128 | 128 | 0.25 | ≤0.063 |
| FDA209P(MSSA) | 0.25 | ≤0.063 | 0.125 | 0.125 | ≤0.063 | ≤0.063 | >64 | 8 |

As is clear from the results of Table 1-2, it was confirmed that the quinolone resistance became higher sequentially from the first-step quinolone-resistant mutants to the fourth-step quinolone-resistant mutant. The quinolones other than SPFX were found to exhibit lower antibacterial activities for the first-step quinolone-resistant mutants. The antibacterial activity of SPFX was found to be lower for the second-step quinolone-resistant mutants. From these results, it was confirmed that the mutation of topoisomerase IV or DNA gyrase allowed bacteria to be resistant to the quinolones.

In contrast, the nybomycins were found to exhibit low antibacterial activities against the bacterial strains against which the quinolones exhibited high antibacterial activities, while the nybomycins were found to exhibit high antibacterial activities against the bacterial strains against which the quinolones exhibited low antibacterial activities. Also, it was confirmed that both the nybomycins acted on DNA gyrase whose Ser84 was substituted by Leu.

Thus, it was suggested that the nybomycins acted specifically on the altered DNA gyrase to exhibit high antibacterial activities against bacterial strains that acquired resistance to the quinolones. Also, through comparison between NM and DNM, the antibacterial activity of DNM was higher than that of NM. Furthermore, from the result of DNM against MS5952-3, the nybomycin was found to have a higher antibacterial activity against the mutation of topoisomerase IV as compared with the quinolones.

(Test Example 4: Evaluation of antibacterial activity of glycopeptides, β-lactams, quinolones and nybomycins against Staphylococcus aureus)

Glycopeptides, imipenem, quinolones and nybomycins were studied for antibacterial activity against the bacterial strains listed in Table 2-1; i.e., MRSA, CA-MRSA and MSSA of Staphylococcus aureus.

Notably, the bacterial strains listed in Table 2-1 are described in the following references, and are available from the corresponding research institutes or NARSA (Network on Antimicrobial Resistance in Staphylococcus aureus) which is a public bacterial strain bank. Each bacterial strain corresponds to each reference as shown in Table 2-1.

Reference 1: Schuhardt VT et al., J Bacteriol, 1968, Sep 96 (3), 734-7
Reference 2: Approved Standard-8th Edition. Wayne, PA: Clinical and Laboratory Standards Institute; CLSI M7-A7.
Reference 3: Aminaka M et al., 2008, Vol 56 (1), 16-20
Reference 4: Kuroda M et al., Lancet, 2001, Apr 21, 357(9264), 1225-40.
Reference 5: Baba T et al., Lancet, 2002, May 25, 359(9320), 1819-27
Reference 6: Novick RP et al., J Bacteriol, 1965, Aug 90, 467-80
Reference 7: Gill SR et al., J Bacteriol, 2005 Apr, 187(7), 2426-38.
Reference 8: Diep BA et al., Lancet, 2006, Mar 4, 367(9512), 731-9.

Here, the glycopeptides used were vancomycin (hereinafter may be referred to as "VCM") and teicoplanin (hereinafter may be referred to as "TEIC").

The β-lactams used were imipenem (hereinafter may be referred to as "IPM") and OXA.

The quinolones used were NFLX, LVFX, OFLX, CPFX, TFLX and SPFX.

The nybomycins used were NM and DNM purified in Test Example 2.

<Measurement of MIC>

The procedure of Test Example 3 was repeated, except that the bacterial strains listed in Table 1-1 were changed to those listed in Table 2-1 and that the compounds allowed to act on the bacterial strains were changed from the compounds shown in Table 1-2 to the compounds shown in Table 2-2, to thereby perform culturing of the bacterial strains, preparation of each compound, and measurement and evaluation of MIC. The results are shown in Table 2-2.

Table 2-1

| Name of bacterial strain | Category | Reference | | Name of bacterial strain | Category | Reference |
|---|---|---|---|---|---|---|
| ATCC29213 | MSSA | 1 | | ISA35-1 | MRSA | 3 |
| FDA209P | MSSA | 2 | | ISA56-3 | MRSA | 3 |
| ISA49-1 | MSSA | 3 | | KBSA34 | MRSA | 3 |
| ISA73-1 | MSSA | 3 | | COL | MRSA | 7 |
| KBSA32 | MRSA | 3 | | ISA58-1 | MRSA | 3 |
| ISA62-1 | MRSA | 3 | | KSA31 | MRSA | 3 |
| N315 | MRSA | 4 | | USA300 FPR3757 | CA-MRSA | 8 |
| MW2 | CA-MRSA | 5 | | KBSA96 | MRSA | 3 |
| NCTC8325 | MSSA | 6 | | KSA24 | MRSA | 3 |
| ISA51-1 | MSSA | 3 | | KBSA171 | MRSA | 3 |

(continued)

| Name of bacterial strain | Category | Reference | | Name of bacterial strain | Category | Reference |
|---|---|---|---|---|---|---|
| ISA37-1 | MSSA | 3 | | KBSA72 | MRSA | 3 |
| ISA12-1 | MRSA | 3 | | KSA36 | MRSA | 3 |
| ISA12-2 | MRSA | 3 | | KBSA56 | MRSA | 3 |
| NARSA(http://www.narsa.net/) | | | | | | |

Table 2-2

| Name of bacterial strain | MIC (mg/L) | | | | | | | | | | | |
|---|---|---|---|---|---|---|---|---|---|---|---|---|
| | Glycopeptides | | $\beta$-Lactams | | Quinolones | | | | | | Nibomycins | |
| | VCM | TEIC | IPM | OXA | NFLX | LVFX | OFLX | CPFX | TFLX | SPFX | NM | DNM |
| ATCC 29213 | 0.5 | 2 | <0.03 | 0.25 | 1 | 0.125 | 0.25 | 0.25 | ≦0.063 | ≦0.063 | 64 | 16 |
| FDA209P | 0.5 | 0.06 | <0.03 | ≦0.063 | 0.25 | ≦0.063 | 0.125 | 0.125 | ≦0.063 | ≦0.063 | 64 | 8 |
| ISA49-1 | 1 | 0.5 | 0.25 | 1 | 1 | 0.25 | 0.25 | 0.25 | ≦0.063 | ≦0.063 | >64 | 16 |
| ISA73-1 | 1 | 2 | 0.25 | 1 | 1 | 0.125 | 0.25 | 0.5 | ≦0.063 | ≦0.063 | >64 | >64 |
| KBSA32 | 0.5 | 1 | 0.25 | 8 | 0.5 | 0.125 | 0.25 | 0.125 | ≦0.063 | ≦0.063 | >64 | 32 |
| ISA62-1 | 1 | 1 | 2 | 8 | 1 | 0.25 | 0.25 | 0.25 | ≦0.063 | ≦0.063 | >64 | >64 |
| N315 | 0.5 | 0.25 | 8 | 32 | 1 | 0.125 | 0.25 | 0.25 | ≦0.063 | ≦0.063 | 64 | 32 |
| MW2 | 0.5 | 0.5 | 2 | 8 | 0.5 | 0.125 | 0.5 | 0.25 | ≦0.063 | ≦0.063 | 64 | 64 |
| NCTC 8325 | 1 | 0.5 | ≦0.063 | 1 | 1 | 0.25 | 0.25 | 0.25 | ≦0.063 | 0.125 | >64 | >64 |
| ISA51-1 | 1 | 0.5 | 0.25 | 1 | 0.5 | 0.25 | 0.5 | 0.5 | ≦0.063 | 0.125 | >64 | 8 |
| ISA37-1 | 1 | 1 | 0.125 | 2 | 1 | 0.25 | 0.25 | 0.25 | ≦0.063 | 0.125 | >64 | >64 |
| ISA12-1 | 0.5 | 1 | 16 | 4 | 1 | 0.25 | 0.5 | 0.5 | ≦0.063 | 0.125 | >64 | 32 |
| ISA12-2 | 0.5 | 1 | 16 | 4 | 1 | 0.25 | 0.5 | 0.25 | ≦0.063 | 0.125 | >64 | 16 |
| ISA35-1 | 1 | 2 | 1 | 4 | 16 | 1 | 2 | 4 | 0.25 | 0.125 | >64 | 64 |
| ISA56-3 | 2 | 4 | 16 | 8 | 0.25 | 0.25 | 0.5 | 0.125 | ≦0.063 | 0.125 | >64 | >64 |
| KBSA34 | 1 | 1 | 0.25 | 16 | 1 | 0.25 | 0.5 | 0.5 | ≦0.063 | 0.125 | >64 | >64 |
| COL | 0.5 | 2 | 64 | >16 | 1 | 0.5 | 0.5 | 0.25 | 0.125 | 0.25 | >64 | >64 |
| ISA58-1 | 1 | 2 | 0.125 | 4 | 1 | 0.5 | 0.5 | 0.5 | 0.5 | 0.5 | 16 | 8 |
| KSA31 | 1 | 1 | 0.125 | 128 | 32 | 4 | 8 | 8 | 4 | 8 | 1 | 0.25 |
| USA300 FPR3757 | 1 | 1 | 2 | 16 | 32 | 32 | 16 | 16 | 16 | 8 | 0.25 | 0.125 |
| KBSA96 | 1 | 1 | 0.25 | 128 | 128 | 8 | 16 | 32 | 16 | 16 | 0.125 | ≦0.063 |
| KSA24 | 1 | 1 | 0.5 | 128 | 128 | 8 | 32 | 32 | 8 | 16 | 0.5 | 0.125 |
| KBSA171 | 1 | 0.25 | 0.25 | >128 | >128 | 16 | 32 | 64 | 8 | 16 | 0.25 | ≦0.063 |

(continued)

| Name of bacterial strain | MIC (mg/L) | | | | | | | | | | | |
|---|---|---|---|---|---|---|---|---|---|---|---|---|
| | Glycopeptides | | β-Lactams | | Quinolones | | | | | | Nibomycins | |
| | VCM | TEIC | IPM | OXA | NFLX | LVFX | OFLX | CPFX | TFLX | SPFX | NM | DNM |
| KBSA72 | 1 | 1 | 0.125 | 128 | >128 | >128 | >128 | 128 | >128 | 128 | ≦0.063 | ≦0.063 |
| KSA36 | 1 | 1 | 0.5 | >128 | >128 | >128 | >128 | 128 | >128 | 128 | ≦0.063 | ≦0.063 |

From the results shown in Table 2-2, the nybomycins exhibited low antibacterial activities against the bacterial strains against which the quinolones exhibited high antibacterial activities, while the nybomycins exhibited high antibacterial activities against the bacterial strains against which the quinolones exhibited low antibacterial activities. Also, regarding the antibacterial activities of the nybomycins against Staphylococcus aureus, the antibacterial activity of DNM was higher than that of NM.

As compared with the quinolones, the glycopeptides and imipenem exhibited higher antibacterial activities against the bacterial strains against which the nybomycins exhibited high antibacterial activities. However, the antibacterial activities of the glycopeptides and imipenem were lower than those of the nybomycins.

(Test Example 5: Evaluation of antibacterial activity of glycopeptides, quinolones and nybomycins against multiply antibiotic-resistant clinical isolates)

The glycopeptides, β-lactams, quinolones and nybomycins used in Test Example 5 were studied for antibacterial activity against VISA clinical isolates of MRSA (hereinafter may be referred to as "VI-MRSA") and VISA clinical isolates of MSSA (hereinafter may be referred to as "VI-MSSA") derived from various countries of the world shown in Table 3-1 as well as VRSA clinical isolates separated in the U.S. shown in Table 4.

Notably, in Table 3-1, the bacterial strains indicated by "†" are described in Lulitanond, A., et al., J Clin Microbiol, 2009, Jul; 47(7), 2311-6. Epub 2009 Apr 29., the bacterial strains indicated by "*" are described in Cui, L., Ma, X., et al., 2003, J Clin Microbiol 41(1), 5-14., and the bacterial strains indicated by "§" are described in Wang, J. L., Tseng, S. P., et al., 2004, Emerg Infect Dis 10(9), 1702-4. These bacterial strains are available from the corresponding research institutes.

Also, the other bacterial strains shown in Tables 3-1 and 4 are available from NARSA.

Here, the glycopeptides used were VCM and TEIC. The β-lactam used was OXA. The quinolones used were NFLX, LVFX, OFLX, CPFX, TFLX and SPFX. The nybomycins used were NM and DNM purified in Test Example 2.

<Measurement of MIC>

The procedure of Test Example 3 was repeated, except that the bacterial strains listed in Table 1-1 were changed to those listed in Tables 3-1 and 4 and that the compounds allowed to act on the bacterial strains were changed from the compounds shown in Table 1-2 to the compounds shown in Tables 3-2 and 4, to thereby perform culturing of the bacterial strains, preparation of each compound, and measurement and evaluation of MIC. The results are shown in Tables 3-2 and 4.

Table 3-1

| Name of bacterial strain | Separated from | Category | | Name of bacterial | Separated from | Category |
|---|---|---|---|---|---|---|
| SA MER-S20 | France | VI-MSSA | | BR5 | Brazil | VI-MRSA |
| SA MER-S12 | France | VI-MSSA | | HIP08926 | America | VI-MRSA |
| SA MER-S6 | France | VI-MSSA | | HIP09143 | America | VI-MRSA |
| LY-1999-01 | Oman | VI-MSSA | | HIP09740 | America | VI-MRSA |
| P1V44 | Belgium | VI-MSSA | | HIP09735 | America | VI-MRSA |
| HIP13036 | America | VI-MRSA | | HIP09662 | America | VI-MRSA |
| HIP13057 | America | VI-MRSA | | 99/3759-V* | The united Kingdom | VI-MRSA |
| B7(JCSC4852)§ | Taiwan | VI-MRSA | | 99/3700-W* | The united Kingdom | VI-MRSA |
| A7(JCSC4852)§ | Taiwan | VI-MRSA | | JCSC7193† | Thailand | VI-MRSA |
| NRS126 | America | VI-MRSA | | 98141* | France | VI-MRSA |
| HIP06297 | America | VI-MRSA | | Mu3 | Japan | VI-MRSA |
| HIP09313 | America | VI-MRSA | | NRS76 | America | VI-MRSA |
| LY-1999-03 | Oman | VI-MRSA | | 28160* | South African Republic | VI-MRSA |
| HIP09737 | America | VI-MSSA | | BR2* | Brazil | VI-MRSA |
| PC* | America | VI-MRSA | | BR3* | Brazil | VI-MRSA |

(continued)

| Name of bacterial strain | Separated from | Category | | Name of bacterial | Separated from | Category |
|---|---|---|---|---|---|---|
| HIP10267 | America | VI-MRSA | | NJ (HIP5836) | America | VI-MRSA |
| LIM2 | France | VI-MRSA | | IL (HIP7737)* | America | VI-MRSA |
| HIP07930 | America | VI-MRSA | | BR1* | Brazil | VI-MRSA |
| HIP10540 | America | VI-MRSA | | MI (HIP5827) | America | VI-MRSA |
| JCSC7203† | Thailand | VI-MRSA | | AMC11094 | Korea | VI-MRSA |
| HIP06854 | America | VI-MRSA | | NRS118 | America | VI-MRSA |
| HIP07920 | Amercia | VI-MRSA | | HIP12864 | America | VI-MRSA |
| NRS79 | America | VI-MRSA | | JSCS7195† | Thailand | VI-MRSA |
| Mu50 | Japan | VI-MRSA | | HIP09433 | America | VI-MRSA |
| BR4* | Brazil | VI-MRSA | | | | |

Table 3-2

| Name of bacterial strain | MIC (mg/L) | | | | | | | | | | |
|---|---|---|---|---|---|---|---|---|---|---|---|
| | Glycopeptides | | $\beta$-Lactams | Quinolones | | | | | | Nibomycins | |
| | VCM | TEIC | OXA | NFLX | LVFX | OFLX | CPFX | TFLX | SPFX | NM | DNM |
| SA MER-S20 | 4 | 32 | 1 | 0.5 | 0.125 | 0.25 | 0.25 | 1 | 0.125 | >64 | >64 |
| SA MER-S12 | 4 | 16 | 1 | 2 | 0.5 | 0.5 | 0.5 | 0.25 | 0.25 | 64 | >64 |
| SA MER-S6 | 4 | 8 | 1 | 2 | 0.5 | 1 | 1 | 0.25 | 0.25 | 64 | >64 |
| LY-1999-01 | 4 | 8 | 1 | 16 | 4 | 8 | 4 | 8 | 2 | 0.125 | ≦0.063 |
| P1V44 | 2 | 16 | 2 | 4 | 4 | 4 | 4 | 8 | 4 | 1 | 0.5 |
| HIP13036 | 8 | 4 | 32 | 16 | 4 | 8 | 4 | 64 | 4 | 0.5 | 0.25 |
| HIP13057 | 4 | 8 | 4 | 128 | 16 | 32 | 64 | >128 | 8 | 0.25 | 0.125 |
| B7 (JCSC4852) | 4 | 16 | 128 | 64 | 8 | 16 | 16 | 16 | 8 | ≦0.063 | 0.125 |
| A7 (JCSC4842) | 3 | 8 | 128 | 32 | 8 | 16 | 16 | 32 | 8 | 0.125 | ≦0.063 |
| NRS126 | 4 | 2 | 256 | 64 | 4 | 16 | 16 | 128 | 8 | 0.5 | 0.5 |
| HIP06297 | 4 | 8 | 512 | 64 | 4 | 16 | 16 | 128 | 8 | 0.5 | 0.125 |
| HIP09313 | 4 | 4 | 512 | 32 | 8 | 16 | 16 | 128 | 8 | 0.5 | 0.125 |
| LY-1999-03 | 4 | 8 | 1,024 | 64 | 4 | 8 | 16 | 32 | 8 | ≦0.063 | ≦0.063 |
| HIP09737 | 4 | 4 | 1 | >128 | 32 | 64 | >128 | >128 | 16 | 1 | 1 |
| PC | 8 | 4 | 4 | 64 | 8 | 16 | 16 | 128 | 16 | 1 | 0.125 |
| HIP10267 | 4 | 4 | 4 | >128 | 32 | 64 | 128 | >128 | 16 | 2 | 0.25 |
| LIM2 | 2 | 8 | 32 | 128 | 32 | 64 | 64 | >128 | 16 | 0.5 | 0.25 |
| HIP07930 | 4 | 4 | 32 | 128 | 32 | 32 | 64 | >128 | 16 | 1 | 0.5 |
| HIP10540 | 4 | 16 | 64 | >128 | 8 | 32 | 64 | 128 | 16 | 0.25 | ≦0.063 |
| JCSC7203† | 2 | 4 | 128 | 64 | 16 | 16 | 8 | 128 | 16 | 0.16 | 0.16 |
| HIP06854 | 4 | 2 | 128 | 128 | 16 | 16 | 16 | 128 | 16 | 1 | 0.5 |
| HIP07920 | 2 | 2 | 128 | 16 | 8 | 16 | 16 | 4 | 16 | 0.25 | 0.25 |
| NRS79 | 2 | 8 | 256 | >128 | 64 | 128 | >128 | >128 | 16 | 1 | 1 |

(continued)

| Name of bacterial strain | MIC (mg/L) | | | | | | | | | | |
|---|---|---|---|---|---|---|---|---|---|---|---|
| | Glycopeptides | | $\beta$-Lactams | Quinolones | | | | | | Nibomycins | |
| | VCM | TEIC | OXA | NFLX | LVFX | OFLX | CPFX | TFLX | SPFX | NM | DNM |
| Mu50 | 8 | 16 | 512 | 128 | 8 | 16 | 32 | >128 | 16 | 0.25 | 0.25 |
| BR4 | 8 | 8 | 512 | 128 | 8 | 64 | 16 | >128 | 16 | 0.25 | 0.25 |
| BR5 | 8 | 4 | 512 | 128 | 16 | 64 | 16 | >128 | 16 | 0.25 | 0.25 |
| HIP08926 | 4 | 4 | 512 | 64 | 16 | 16 | 16 | 128 | 16 | 0.25 | 0.5 |
| HIP09143 | 4 | 4 | 512 | >128 | 32 | 64 | 64 | >128 | 16 | 0.25 | 0.5 |
| HIP09740 | 1 | 4 | 512 | >128 | 32 | 64 | >128 | >128 | 16 | 0.25 | 0.25 |
| HIP09735 | 4 | 4 | 512 | >128 | 8 | 32 | 64 | >128 | 16 | 0.25 | 0.5 |
| HIP09662 | 4 | 4 | 512 | 64 | 32 | 32 | 32 | >128 | 16 | 1 | 0.25 |
| 99/3759-V | 2 | 8 | 512 | 128 | 16 | 32 | 32 | 16 | 16 | 0.5 | 1 |
| 99/3700-W | 2 | 2 | 512 | 64 | 16 | 16 | 16 | 8 | 16 | 0.25 | 0.25 |
| JCSC7193† | 4 | 8 | >128 | 64 | 16 | 16 | 8 | 128 | 16 | 0.33 | 0.33 |
| 98141 | 2 | 4 | >128 | 64 | 16 | 16 | 32 | 32 | 16 | 0.5 | 0.125 |
| Mu3 | 0.5 | 8 | 512 | 128 | 16 | 16 | 32 | 16 | 16 | 0.5 | 0.5 |
| NRS76 | 2 | 4 | 1 | 128 | 16 | 64 | 64 | >128 | 32 | 0.25 | ≦0.063 |
| 28160 | 4 | 4 | 32 | 64 | 8 | 64 | 4 | 32 | 32 | 0.5 | 0.125 |
| BR2 | 4 | 1 | 64 | 64 | 8 | 64 | 4 | 32 | 32 | 0.25 | 0.125 |
| BR3 | 8 | 4 | 64 | 64 | 16 | 64 | 4 | 32 | 32 | 0.25 | 0.125 |
| NJ (HIP5836) | 8 | 8 | 64 | 128 | 16 | 64 | 16 | 32 | 32 | 0.5 | 0.25 |
| IL(HIP7737) | 4 | 4 | 64 | >128 | 32 | 128 | >128 | >128 | 32 | 1 | 1 |
| BR1 | 2 | 2 | 256 | 128 | 16 | 64 | 16 | 128 | 32 | 0.5 | 0.25 |
| MI (HIP5827) | 8 | 8 | 512 | 128 | 32 | 128 | 16 | 16 | 32 | 0.5 | ≦0.063 |
| AMC11094 | 4 | 8 | 512 | 128 | 16 | 128 | 32 | >128 | 32 | 1 | 0.125 |
| NRS118 | 8 | 8 | 1,024 | 128 | 32 | 64 | 64 | >128 | 32 | 0.25 | 2 |

(continued)

| Name of bacterial strain | MIC (mg/L) | | | | | | | | | | |
|---|---|---|---|---|---|---|---|---|---|---|---|
| | Glycopeptides | | β-Lactams | Quinolones | | | | | | Nibomycins | |
| | VCM | TEIC | OXA | NFLX | LVFX | OFLX | CPFX | TFLX | SPFX | NM | DNM |
| HIP12864 | 4 | 8 | 1,024 | 64 | 32 | 64 | 16 | >128 | 32 | 0.5 | 0.125 |
| JCSC7195† | 1 | 8 | >128 | 128 | 32 | 64 | 64 | >128 | 32 | 0.33 | 0.16 |
| HIP09433 | 2 | 8 | 256 | >128 | 32 | 64 | 128 | >128 | 64 | 1 | 0.5 |

†Reference for strain JCSC7193, 7195, 7203
(J Clin Microbiol. 2009 Jul; 47(7):2311-6. Epub 2009 Apr 29.)

From the results shown in Table 3-2, all the bacterial strains except for three VI-MSSA strains (SA MER-S20, SAMER-S12 and SA MER-S6) exhibited resistance to the quinolones, suggesting that almost all the VISA strains from various countries of the world are resistant to the quinolones.

In contrast, the nybomycins exhibited no antibacterial activity against these three strains to which the quinolones were effective, but exhibited high antibacterial activities against the quinolone-resistant bacterial strains except for the three strains.

Table 4

| Name of bacterial strain | Separated from (in America) | MIC (mg/L) | | | | | | | | | | |
|---|---|---|---|---|---|---|---|---|---|---|---|---|
| | | Glycopeptides | | β-Lactam | Quinolones | | | | | | Nibomycins | |
| | | VCM** | TEIC** | OXA** | NFLX | LVFX | OFLX | CPFX | TFLX | SPFX | NM | DNM |
| VRS1 | Michigan | >256 | 64 | >16 | >128 | 16 | 32 | 128 | 4 | 8 | 0.125 | ≤0.063 |
| VRS2 | Pennsylvania | 64 | 8 | >16 | >128 | 16 | 32 | 128 | >128 | 8 | 0.25 | 0.125 |
| VRS3 | New York | >32 | 16 | >16 | 128 | 16 | 32 | 64 | >128 | 16 | 0.125 | ≤0.063 |
| VRS4 | New York | >32 | 32 | >16 | 128 | 32 | 32 | 64 | >128 | 16 | 0.125 | ≤0.063 |
| VRS5 | Michigan | >256 | 128 | >16 | >128 | >128 | >128 | >128 | >128 | 128 | ≤0.063 | ≤0.063 |
| VRS6 | Michigan | >256 | >256 | >16 | >128 | 32 | 64 | >128 | >128 | 16 | ≤0.063 | ≤0.063 |
| VRS7 | Michigan | >256 | >256 | 8 | >128 | 32 | 64 | 64 | 4 | 16 | 0.5 | 0.125 |

** Based on NARSA(http://www.narsa.net/)

From the results shown in Table 4, the VRSA strains exhibited resistance to all of the glycopeptides, β-lactams and quinolones. In contrast, the nybomycins exhibited high antibacterial activities against these bacterial strains. Notably, these strains were subdivided from NARSA.

(Test Example 6: Evaluation of antibacterial activity of quinolones and nybomycins against enterococcus)

Quinolones and nybomycins were evaluated for antibacterial activity against Enterococcus faecalis shown in Table 6, wherein the other strains than NCTC12201 and NCTC12203 were subdivided from Shionogi & Co., Ltd (note that these strains are available also from the Department of Bacteriology, Juntendo University). The NCTC strains are both vancomycin-resistant E. faecalis and available from the National Collection of Type Cultures (NCTC). Notably, the quinolone-resistant enterococci shown in Table 6 are enterococci having an MIC of 4 mg/L or higher against LVFX.

Here, the quinolones used were NFLX, LVFX, OFLX, CPFX, TFLX and SPFX. The nybomycins used were NM and DNM purified in Test Example 2.

<Measurement of MIC>

The procedure of Test Example 3 was repeated, except that the bacterial strains listed in Table 1-1 were changed to those listed in Table 6 and that the compounds allowed to act on the bacterial strains were changed from the compounds shown in Table 1-2 to the compounds shown in Table 6, to thereby perform culturing of the bacterial strains, preparation of each compound, and measurement and evaluation of MIC. The results are shown in Table 6.

Table 6

| Bacterial strain | | MIC (mg/L) | | | | | | | |
|---|---|---|---|---|---|---|---|---|---|
| | | Quinolones | | | | | | Nibomycins | |
| | | NFLX | LVFX | OFLX | CPFX | TFLX | SPFX | NM | DNM |
| Quinolone-susceptible enterococci | NCTC12201* | 2 | 1 | 2 | 1 | 0.25 | 0.5 | 8 | 16 |
| | NCTC12203* | 2 | 1 | 2 | 1 | 0.5 | 0.5 | 16 | >64 |
| | 36-15672 | 4 | 1 | 2 | 1 | 0.5 | 1 | >64 | >64 |
| | 36-15673 | 4 | 1 | 2 | 1 | 0.5 | 1 | >64 | >64 |
| | 36-15678 | 4 | 1 | 2 | 1 | 0.5 | 0.5 | >64 | >64 |
| | 36-15688 | 2 | 1 | 2 | 1 | ≤0.063 | 0.5 | >64 | >64 |
| | 36-15696 | 4 | 1 | 2 | 1 | 0.25 | 0.5 | >64 | >64 |
| | 36-15720 | 8 | 1 | 2 | 2 | 0.5 | 1 | >64 | >64 |
| | 36-15736 | 4 | 1 | 2 | 1 | 0.25 | 0.5 | >64 | >64 |
| | 36-15718 | 4 | 2 | 2 | 1 | 0.25 | 0.5 | 32 | 32 |
| | 36-15730 | 8 | 2 | 4 | 2 | 0.5 | 1 | >64 | 64 |
| | 36-15741 | 4 | 2 | 4 | 2 | 0.5 | 1 | >64 | >64 |
| Quinolone-resistant enterococci | 36-15716 | >128 | 32 | 128 | 128 | >128 | 64 | 1 | 4 |
| | 36-15703 | 64 | 32 | 64 | 32 | >128 | 32 | 2 | 0.25 |
| | 36-15686 | >128 | 64 | 128 | 128 | >128 | 128 | 0.5 | 2 |
| | 36-15726 | 128 | 64 | 128 | 64 | >128 | 64 | 0.5 | 2 |
| | 36-15694 | >128 | 64 | 64 | 32 | >128 | 64 | 1 | 2 |
| | 36-15722 | 128 | 64 | 64 | 64 | >128 | 64 | 1 | 4 |
| *Vancomycin-resistant E. faecalis; VRE | | | | | | | | | |

From the results shown in Table 6, the nybomycins exhibited a low antibacterial activity against the quinolone-susceptible bacteria while exhibited a high antibacterial activity against the quinolone-resistant bacteria. Also, although

DNM exhibited a higher antibacterial activity against Staphylococcus aureus than NM in Test Example 4, NM exhibited a higher antibacterial activity against enterococci than DNM.

As suggested from the results of Test Examples 4 to 6, the nybomycins can be suitably used for the treatment of infectious diseases caused by quinolone-resistant bacteria, since they are ineffective to bacteria susceptible to quinolones conventionally used as antibacterial agents not to permit the quinolone-susceptible bacteria to be resistant as well as they are effective to the quinolone-resistant bacteria; i.e., exhibit an excellent antibacterial activity against bacteria to which the quinolones have conventionally been ineffective. Furthermore, use of the nybomycins in combination with the conventionally used quinolones can effectively treat infectious diseases regardless of whether they are caused by quinolone-susceptible bacteria or quinolone-resistant bacteria.

The antibacterial agent for use in treating an individual infected with a drug-resistant bacteria, the infectious disease therapeutic drug for use in treating an individual infected with a drug-resistant bacteria containing the antibacterial agent for drug-resistant bacteria, and the infectious disease combined therapeutic drug for use in treating an individual infected with a drug-restistant bacteria containing the antibacterial agent for drug-resistant bacteria and conventionally used quinolones according to the present invention exhibit a low antibacterial activity against or are ineffective to drug-susceptible bacteria including at least quinolone-susceptible bacteria not to permit the drug-susceptible bacteria to be drug resistant; exhibit an excellent antibacterial activity against drug-resistant bacteria resistant to at least one drug; involve no side effects; and can be produced simply. Thus, they are used for the prevention or treatment for infectious diseases caused by drug-resistant bacteria, especially infectious diseases caused by quinolone-resistant bacteria.

## Claims

1. An antibacterial agent for use in treating an individual infected with a drug-resistant bacteria, wherein the antibacterial agent is a compound represented by the following General Formula (1):

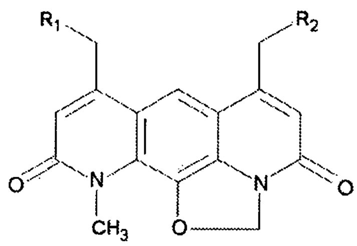.

General Formula (1)

where $R_1$ represents OH or H and $R_2$ represents H, and wherein the antibacterial agent for drug-resistant bacteria is effective to drug-resistant bacteria resistant to at least one drug.

2. The antibacterial agent for use in treating an individual infected with the drug-resistant bacteria according to claim 1, wherein the drug-resistant bacteria include at least quinolone-resistant bacteria.

3. The antibacterial agent for use in treating an individual infected with the drug-resistant bacteria according to claim 1 or 2, wherein the antibacterial agent for drug-resistant bacteria is ineffective to at least quinolone-susceptible bacteria.

4. An infectious disease therapeutic drug for use in treating an individual infected with a drug-resistant bacteria comprising:

an antibacterial agent for drug-resistant bacteria, wherein the antibacterial agent for drug-resistant bacteria comprises:

a compound represented by the following General Formula (I):

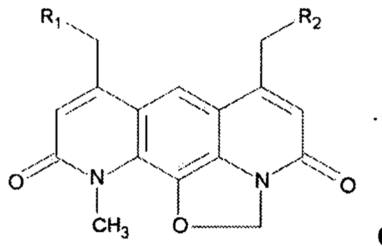

General Formula (1)

where $R_1$ represents OH or H and $R_2$ represents H, and
wherein the antibacterial agent for drug-resistant bacteria is effective to drug-resistant bacteria resistant to at least one drug.

5. The infectious disease therapeutic drug for use in treating an individual infected with the drug-resistant bacteria according to claim 4, wherein the infectious disease therapeutic drug is at least one of a MRSA infectious disease therapeutic drug, a VISA infectious disease therapeutic drug and a VRSA infectious disease therapeutic drug.

6. The infectious disease therapeutic drug for use in treating an individual infected with the drug-resistant bacteria according to claim 4, wherein the infectious disease therapeutic drug is an enterococcus infectious disease therapeutic drug.

7. The infectious disease therapeutic drug for use in treating an individual infected with the drug-resistant bacteria according to any one of claims 4 to 6, wherein the drug-resistant bacteria include at least quinolone-resistant bacteria.

8. The infectious disease therapeutic drug for use in treating an individual infected with the drug-resistant bacteria according to any one of claims 4 to 7, wherein the antibacterial agent for drug-resistant bacteria is ineffective to at least quinolone-susceptible bacteria.

9. An infectious disease combined drug for use in treating an individual infected with a drug-resistant bacteria comprising:

an antibacterial agent for drug-resistant bacteria, and
at least one quinolone,
wherein the antibacterial agent for drug-resistant bacteria comprises:

a compound represented by the following General Formula (I):

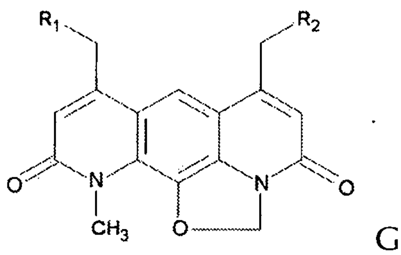

General Formula (1)

where $R_1$ represents OH or H and $R_2$ represents H, and
wherein the antibacterial agent for drug-resistant bacteria is effective to drug-resistant bacteria resistant to at least one drug.

10. The infectious disease combined drug for use in treating an individual infected with the drug-resistant bacteria according to claim 9, wherein the drug-resistant bacteria include at least quinolone-resistant bacteria.

11. The infectious disease combined drug for use in treating an individual infected with the drug-resistant bacteria according to claim 9 or 10, wherein the antibacterial agent for drug-resistant bacteria is ineffective to at least quinolone-susceptible bacteria.

**Patentansprüche**

1. Antibakterielles Mittel zur Verwendung bei der Behandlung eines Individuums, das mit arzneimittelresistenten Bakterien infiziert ist,
wobei das antibakterielle Mittel eine Verbindung ist, die durch die folgende allgemeine Formel (1) dargestellt wird:

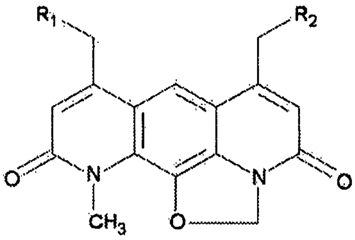

Allgemeine Formel (1)

wobei R, OH oder H darstellt und $R_2$ H darstellt, und
wobei das antibakterielle Mittel für arzneimittelresistente Bakterien gegenüber arzneimittelresistenten Bakterien wirksam ist, die gegenüber mindestens einem Arzneimittel resistent sind.

2. Antibakterielles Mittel zur Verwendung bei der Behandlung eines Individuums, das mit arzneimittelresistenten Bakterien infiziert ist, gemäß Anspruch 1, wobei die arzneimittelresistenten Bakterien mindestens Chinolon-resistente Bakterien einschließen.

3. Antibakterielles Mittel zur Verwendung bei der Behandlung eines Individuums, das mit arzneimittelresistenten Bakterien infiziert ist, gemäß Anspruch 1 oder 2, wobei das antibakterielle Mittel für arzneimittelresistente Bakterien mindestens gegenüber Chinolon-empfindlichen Bakterien unwirksam ist.

4. Infektionskrankheits-therapeutisches Arzneimittel zur Verwendung bei der Behandlung eines Individuums, das mit arzneimittelresistenten Bakterien infiziert ist, umfassend:

   ein antibakterielles Mittel für arzneimittelresistente Bakterien,
   wobei das antibakterielle Mittel für arzneimittelresistente Bakterien umfasst:

      eine Verbindung, die durch die folgende Allgemeine Formel (I) dargestellt ist:

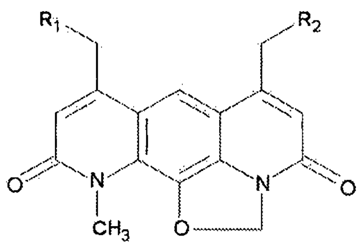

Allgemeine Formel (1)

   wobei $R_1$ OH oder H darstellt und $R_2$ H darstellt, und
   wobei das antibakterielle Mittel für arzneimittelresistente Bakterien gegenüber arzneimittelresistenten Bakterien wirksam ist, die gegenüber mindestens einem Arzneimittel resistent sind.

5. Infektionskrankheits-therapeutisches Arzneimittel zur Verwendung bei der Behandlung eines Individuums, das mit arzneimittelresistenten Bakterien infiziert ist, gemäß Anspruch 4, wobei das infektionskrankheits-therapeutische Arzneimittel mindestens eines von einem MRSA-infektionskrankheits-therapeutischem Mittel, einem VISA-infektionskrankheits-therapeutischem Arzneimittel und einem VRSA-infektionskrankheits-therapeutischem Mittel ist.

6. Infektionskrankheits-therapeutisches Arzneimittel zur Verwendung bei der Behandlung eines Individuums, das mit

arzneimittelresistenten Bakterien infiziert ist, gemäß Anspruch 4, wobei das infektionskrankheits-therapeutische Arzneimittel ein Enterococcus-infektionskrankheits-therapeutisches Arzneimittel ist.

**7.** Infektionskrankheits-therapeutisches Arzneimittel zur Verwendung bei der Behandlung eines Individuums, das mit arzneimittelresistenten Bakterien infiziert ist, gemäß einem der Ansprüche 4 - 6, wobei die arzneimittelresistenten Bakterien mindestens Chinolon-resistente Bakterien einschließen.

**8.** Infektionskrankheits-therapeutisches Arzneimittel zur Verwendung bei der Behandlung eines Individuums, das mit arzneimittelresistenten Bakterien infiziert ist, gemäß einem der Ansprüche 4 - 7, wobei das antibakterielle Mittel für arzneimittelresistente Bakterien gegenüber mindestens Chinolon-empfindlichen Bakterien unwirksam ist.

**9.** Infektionskrankheits-Kombinationsarzneimittel zur Verwendung bei der Behandlung eines Individuums, das mit arzneimittelresistenten Bakterien infiziert ist, umfassend:

ein antibakterielles Mittel für arzneimittelresistente Bakterien, und
mindestens ein Chinolon,
wobei das antibakterielle Mittel für arzneimittelresistente Bakterien umfasst:

eine Verbindung, die durch die folgende Allgemeine Formel (I) dargestellt wird:

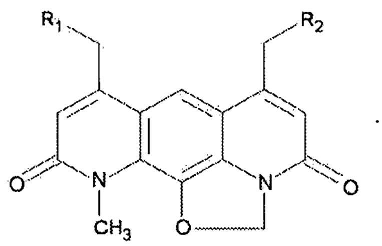

Allgemeine Formel (1)

wobei $R_1$ OH oder H darstellt und $R_2$ H darstellt, und
wobei das antibakterielle Mittel für arzneimittelresistente Bakterien gegenüber arzneimittelresistenten Bakterien wirksam ist, die gegenüber mindestens einem Arzneimittel resistent sind.

**10.** Infektionskrankheits-Kombinationsarzneimittel zur Verwendung bei der Behandlung eines Individuums, das mit arzneimittelresistenten Bakterien infiziert ist, gemäß Anspruch 9, wobei die arzneimittelresistenten Bakterien mindestens Chinolon-resistente Bakterien einschließen.

**11.** Infektionskrankheits-Kombinationsarzneimittel zur Verwendung bei der Behandlung eines Individuums, das mit arzneimittelresistenten Bakterien infiziert ist, gemäß Anspruch 9 oder 10, wobei das antibakterielle Mittel für arzneimittelresistente Bakterien gegenüber mindestens Chinolon-empfindlichen Bakterien unwirksam ist.

## Revendications

**1.** Agent antibactérien pour utilisation dans le traitement d'un individu infecté par une bactérie résistante aux médicaments,
lequel agent antibactérien est un composé représenté par la formule générale (1) :

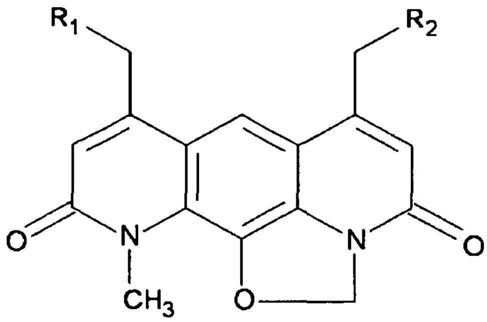

Formule générale (1)

dans laquelle $R_1$ représente OH ou H et $R_2$ représente H, et lequel agent antibactérien pour bactéries résistantes aux médicaments est efficace contre les bactéries résistantes aux médicaments qui résistent à au moins un médicament.

2. Agent antibactérien pour utilisation dans le traitement d'un individu infecté par une bactérie résistante aux médicaments selon la revendication 1, dans lequel la bactérie résistante aux médicaments englobe au moins une bactérie résistante aux quinolones.

3. Agent antibactérien pour utilisation dans le traitement d'un individu infecté par une bactérie résistante aux médicaments selon la revendication 1 ou 2, lequel agent antibactérien pour bactéries résistantes aux médicaments est inefficace contre au moins une bactérie sensible aux quinolones.

4. Médicament thérapeutique contre une maladie infectieuse pour utilisation dans le traitement d'un individu infecté par une bactérie résistante aux médicaments comprenant :

un agent antibactérien pour bactéries résistantes aux médicaments, dans lequel l'agent antibactérien pour bactéries résistantes aux médicaments comprend :

un composé représenté par la formule générale (1) :

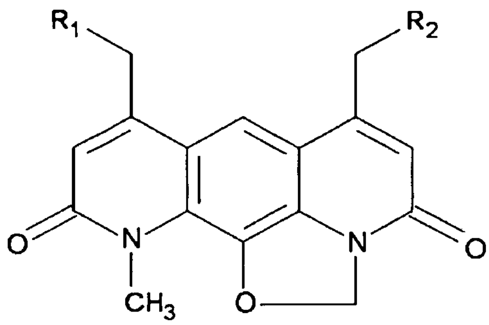

Formule générale (1)

dans laquelle $R_1$ représente OH ou H et $R_2$ représente H, et dans lequel l'agent antibactérien pour bactéries résistantes aux médicaments est efficace contre les bactéries résistantes aux médicaments qui résistent à au moins un médicament.

5. Médicament thérapeutique contre une maladie infectieuse pour utilisation dans le traitement d'un individu infecté par une bactérie résistante aux médicaments selon la revendication 4, lequel médicament thérapeutique contre une maladie infectieuse est au moins l'un parmi un médicament thérapeutique contre une maladie infectieuse à SARM, un médicament thérapeutique contre une maladie infectieuse à SAIV et un médicament thérapeutique contre une maladie infectieuse à SARV.

6. Médicament thérapeutique contre une maladie infectieuse pour utilisation dans le traitement d'un individu infecté par une bactérie résistante aux médicaments selon la revendication 4, lequel médicament thérapeutique contre une maladie infectieuse est un médicament thérapeutique contre une maladie infectieuse à entérocoques.

7. Médicament thérapeutique contre une maladie infectieuse pour utilisation dans le traitement d'un individu infecté par une bactérie résistante aux médicaments selon l'une quelconque des revendications 4 à 6, dans lequel la bactérie résistante aux médicaments englobe au moins une bactérie résistante aux quinolones.

8. Médicament thérapeutique contre une maladie infectieuse pour utilisation dans le traitement d'un individu infecté par une bactérie résistante aux médicaments selon l'une quelconque des revendications 4 à 7, dans lequel l'agent antibactérien pour bactéries résistantes aux médicaments est inefficace contre au moins une bactérie sensible aux quinolones.

9. Médicament combiné contre une maladie infectieuse pour utilisation dans le traitement d'un individu infecté par une bactérie résistante aux médicaments comprenant :

un agent antibactérien pour bactéries résistantes aux médicaments, et
au moins une quinolone,
dans lequel l'agent antibactérien pour bactéries résistantes aux médicaments comprend :

un composé représenté par la formule générale (1) :

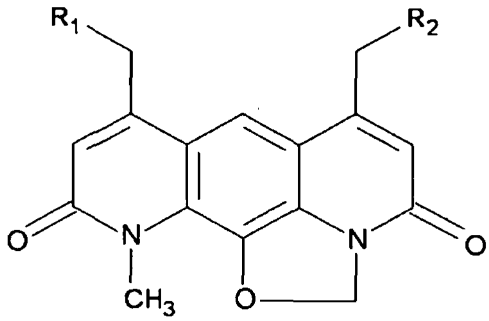

Formule générale (1)

dans laquelle $R_1$ représente OH ou H et $R_2$ représente H, et
dans lequel l'agent antibactérien pour bactéries résistantes aux médicaments est efficace contre les bactéries résistantes aux médicaments qui résistent à au moins un médicament.

10. Médicament combiné contre une maladie infectieuse pour utilisation dans le traitement d'un individu infecté par une bactérie résistante aux médicaments selon la revendication 9, dans lequel la bactérie résistante aux médicaments englobe au moins une bactérie résistante aux quinolones.

11. Médicament combiné contre une maladie infectieuse pour utilisation dans le traitement d'un individu infecté par une bactérie résistante aux médicaments selon la revendication 9 ou 10, dans lequel l'agent antibactérien pour bactéries résistantes aux médicaments est inefficace contre au moins une bactérie sensible aux quinolones.

FIG. 1

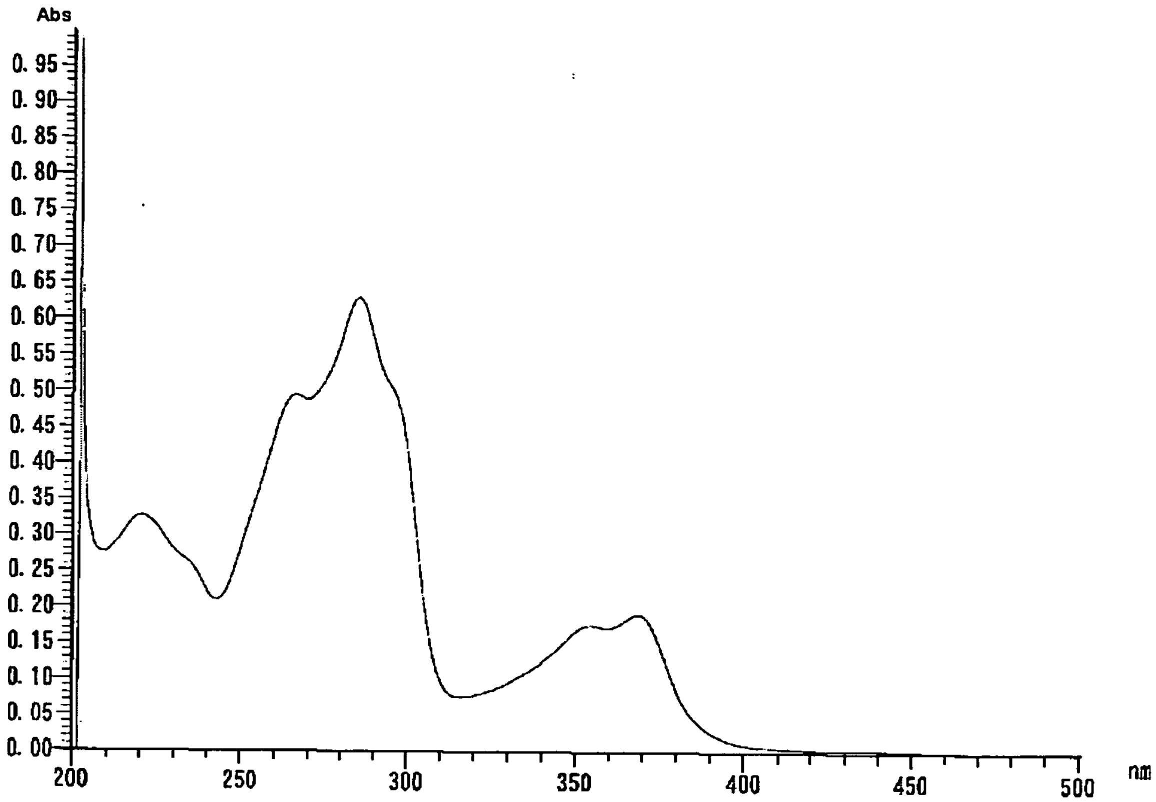
Abs
0. 95
0. 90
0. 85
0. 80
0. 75
0. 70
0. 65
0. 60
0. 55
0. 50
0. 45
0. 40
0. 35
0. 30
0. 25
0. 20
0. 15
0. 10
0. 05
0. 00
200
250
300
350
400
450
500
nm

FIG. 2

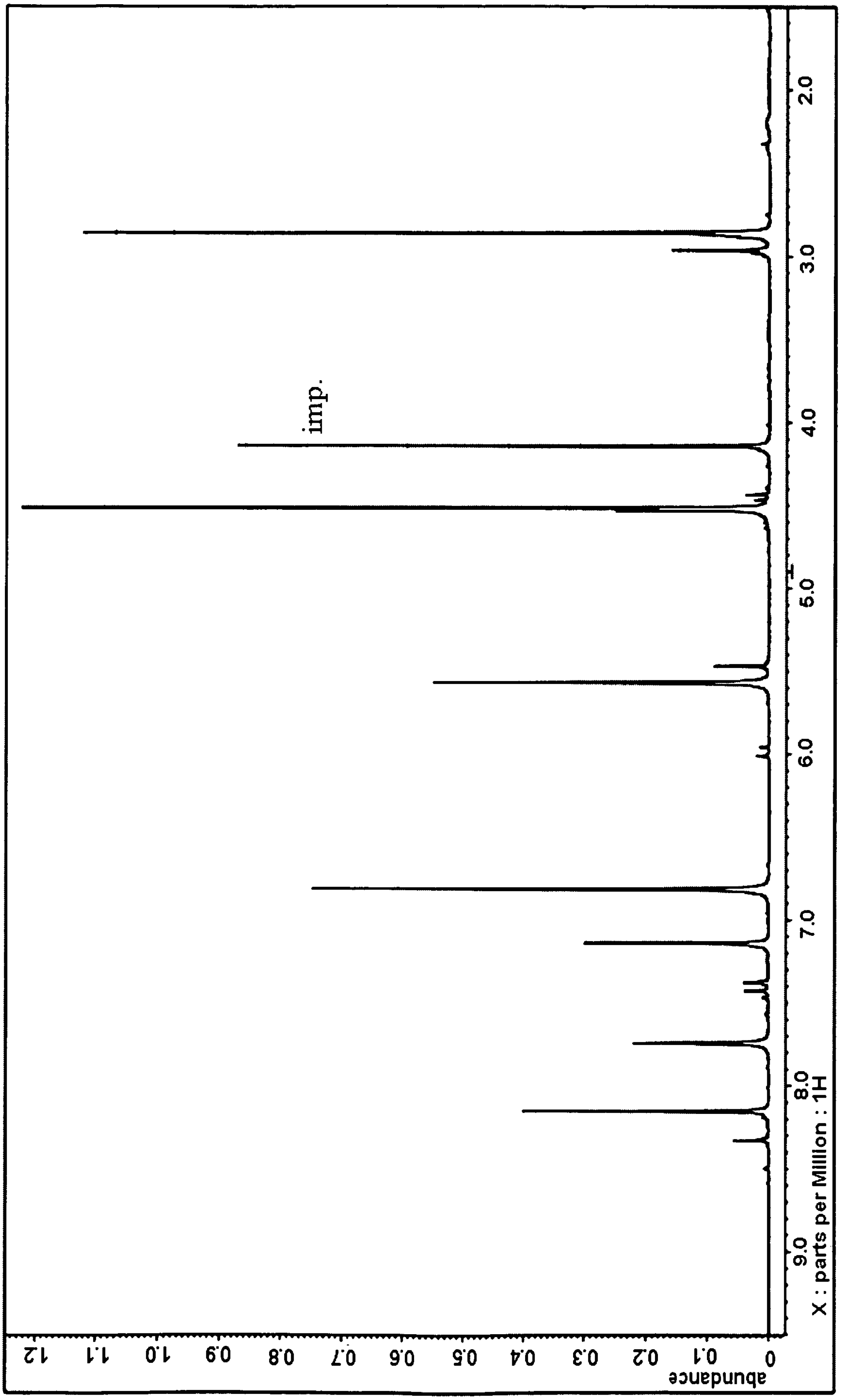
imp.
X : parts per Million : 1H
abundance
9.0
8.0
7.0
6.0
5.0
4.0
3.0
2.0
0
0.1
0.2
0.3
0.4
0.5
0.6
0.7
0.8
0.9
1.0
1.1
1.2

FIG. 3

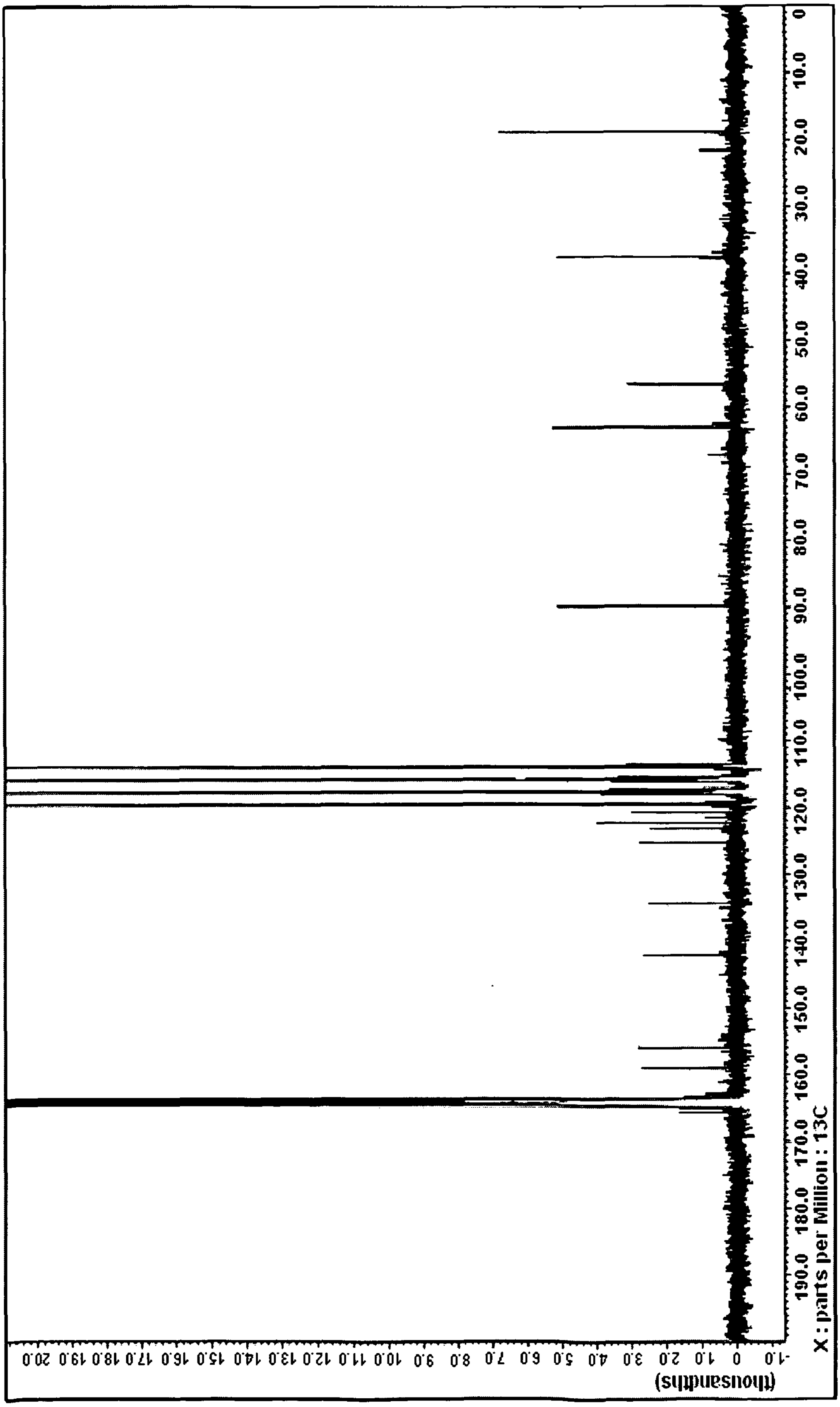

FIG. 4

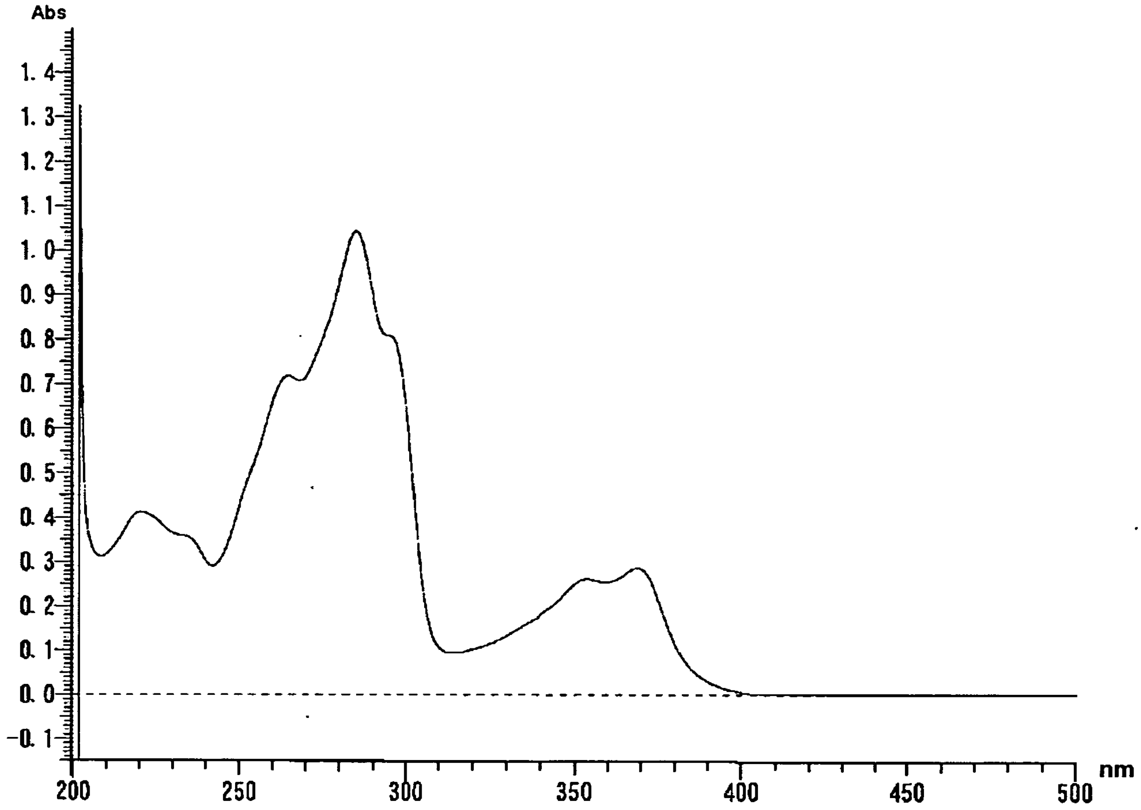
Abs
1.4
1.3
1.2
1.1
1.0
0.9
0.8
0.7
0.6
0.5
0.4
0.3
0.2
0.1
0.0
-0.1
200
250
300
350
400
450
500
nm

FIG. 5

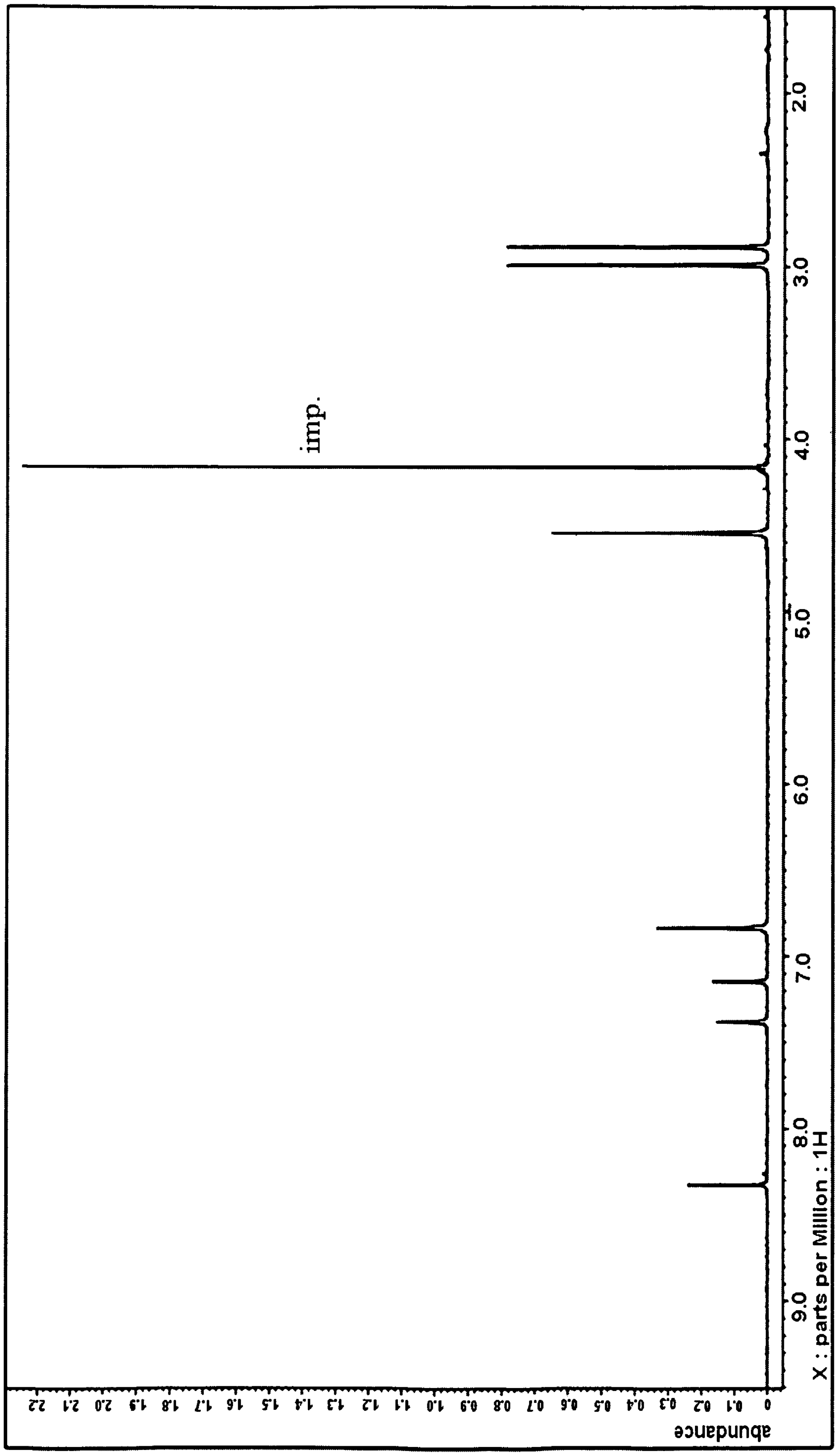
imp.
2.0
3.0
4.0
5.0
6.0
7.0
8.0
9.0
X : parts per Million : 1H
abundance
0 0.1 0.2 0.3 0.4 0.5 0.6 0.7 0.8 0.9 1.0 1.1 1.2 1.3 1.4 1.5 1.6 1.7 1.8 1.9 2.0 2.1 2.2

FIG. 6

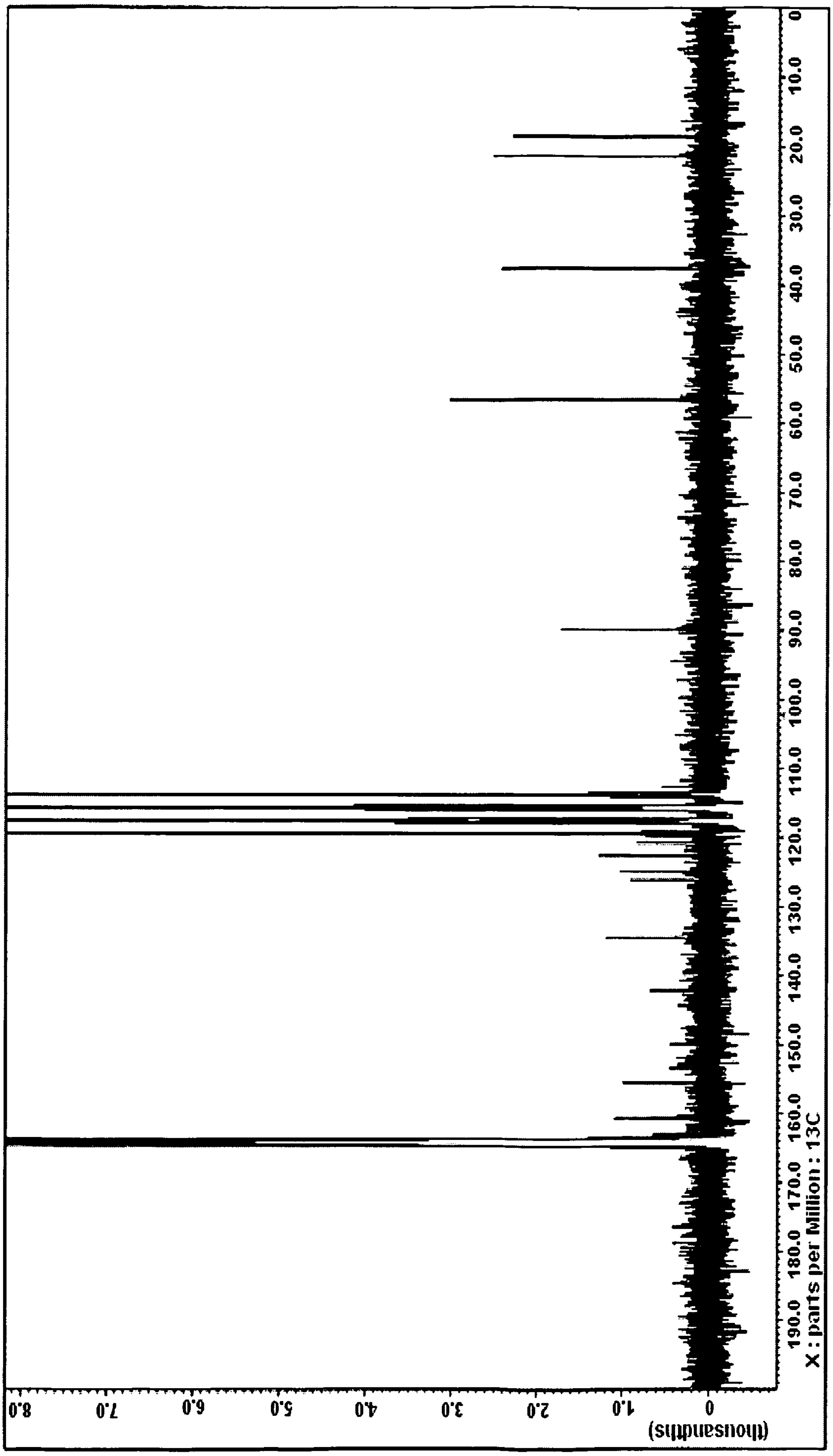
0
10.0
20.0
30.0
40.0
50.0
60.0
70.0
80.0
90.0
100.0
110.0
120.0
130.0
140.0
150.0
160.0
170.0
180.0
190.0
X : parts per Million : 13C
0
1.0
2.0
3.0
4.0
5.0
6.0
7.0
8.0
(thousandths)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Frontiers in Antimicrobial Resistance. ASM Press American Society for Microbiology, 2005
- **JEVONS, M. P.** *Br Med J.s,* 1961, vol. 1, 124-125
- **HIRAMATSU, K. ; HANAKI, H. et al.** *J Antimicrob Chemother,* 1997, vol. 40 (1), 135-6
- **PERICHON B ; COURVALIN P.** *Antimicrob Agents Chemother,* 08 June 2009, vol. 53 (11), 4580-7
- **NADZAH, A.M. et al.** *The Journal of Antibiotics,* 1977, vol. 30 (6), 523-524
- **STRELITZ F et al.** *PNAS,* 1995, vol. 41 (9), 620-624
- **RINEHART KL JR et al.** *J Am Chem Soc,* 1970, vol. 92 (23), 6994-6995
- **NAGANAWA H et al.** *J Antibiot,* 1970, vol. 23 (7), 365-368
- **PÉRICHON B ; COURVALIN P.** *Antimicrob Agents Chemother,* 08 June 2009, vol. 53 (11), 4580-7
- **DIEP BA ; GILL SR ; CHANG RF ; PHAN TH ; CHEN JH ; DAVIDSON MG ; LIN F ; LIN J ; CARLETON HA ; MONGODIN EF.** Perdreau-Remington F. Complete genome sequence of USA300, an epidemic clone of community-acquired meticillin-resistant Staphylococcus aureus. *Lancet.,* 04 March 2006, vol. 367 (9512), 731-9
- **FUKUDA, H. ; HORI, S. et al.** Antibacterial activity of gatifloxacin (AM-1155, CG5501, BMS-206584), a newly developed fluoroquinolone, against sequentially acquired quinolone-resistant mutants and the norA transformant of Staphylococcus aureus. *Antimicrob Agents Chemother,* 1998, vol. 42 (8), 1917-22
- **SCHUHARDT VT et al.** *J Bacteriol,* 09 September 1968, vol. 6 (3), 734-7
- **KURODA et al.** *Lancet,* 21 April 2001, vol. 357 (9264), 1225-40
- **BABA T et al.** *Lancet,* 25 May 2002, vol. 359 (9320), 1819-27
- **NOVICK RP et al.** *J Bacteriol,* 09 August 1965, vol. 0, 467-80
- **GILL SR et al.** *J Bacteriol,* 18 April 2005, vol. 7 (7), 2426-38
- **BA et al.** *Lancet,* 04 March 2006, vol. 367 (9512), 731-9
- **LULITANOND, A. et al.** *J Clin Microbiol,* 29 April 2009, vol. 47 (7), 2311-6
- **CUI, L. ; MA, X. et al.** *J Clin Microbiol,* 2003, vol. 41 (1), 5-14
- **WANG, J. L. ; TSENG, S. P. et al.** *Emerg Infect Dis,* 2004, vol. 10 (9), 1702-4
- *J Clin Microbiol.,* 29 April 2009, vol. 47 (7), 2311-6